# EUROPÄISCHE PATENTSCHRIFT

(11) **EP 1 554 234 B1**
(45) Veröffentlichungstag und Bekanntmachung des Hinweises auf die Patenterteilung: **09.08.2006**
(21) Anmeldenummer: 03808708.6
(22) Anmeldetag: 06.10.2003
(51) Int. Cl.: C07C 51/44, C07C 57/04

(54) **VERFAHREN ZUR HERSTELLUNG VON ACRYLSÄURE**
METHOD FOR THE PRODUCTION OF ACRYLIC ACID
PROCEDE DE PRODUCTION D'ACIDE ACRYLIQUE

(30) Priorität: 10.10.2002 DE 10247240
(43) Veröffentlichungstag der Anmeldung: 20.07.2005
(73) Patentinhaber: BASF Aktiengesellschaft, 67056 Ludwigshafen (DE)
(72) Erfinder: THIEL, Joachim, 67435 Neustadt (DE); HAMMON, Ulrich, 68163 Mannheim (DE); BAUMANN, Dieter, 67227 Frankenthal (DE); HEILEK, Jörg, 69245 Bammental (DE); SCHRÖDER, Jürgen, 67071 Ludwigshafen (DE); MÜLLER-ENGEL, Klaus Joachim, 76297 Stutensee (DE)
(86) Internationale Anmeldenummer: PCT/EP2003/011015
(87) Internationale Veröffentlichungsnummer: WO 2004/035514

(56) Entgegenhaltungen:
- WO-A-01/77056
- DE-A- 19 924 532
- DE-A- 19 924 533

## Beschreibung

Vorliegende Erfindung betrifft ein Verfahren zur Herstellung von Acrylsäure, bei dem man durch heterogen katalysierte Gasphasen-Partialoxidation wenigstens eines C₃-Vorläufers der Acrylsäure mit molekularem Sauerstoff an im festen Aggregatzustand befindlichen Katalysatoren bei erhöhter Temperatur ein Acrylsäure enthaltendes Produktgasgemisch erzeugt, die Temperatur des heißen, Acrylsäure enthaltenden Produktgasgemisches durch direkte Kühlung mit einer Quenchflüssigkeit 1 zunächst verringert und anschließend das abgekühlte, gegebenenfalls Teile an verdampfter Quenchflüssigkeit 1 enthaltende, Produktgasgemisch in eine mit trennwirksamen Einbauten ausgerüstete Kondensationskolonne leitet, innerhalb der Kondensationskolonne in sich selbst aufsteigen lässt, dabei fraktionierend kondensiert sowie im Seitenabzug als Zielprodukt eine rohe Acrylsäure aus der Kondensationskolonne entnimmt und aus dem Sumpf der Kondensationskolonne Acrylsäure-Oligomere enthaltende Sumpfflüssigkeit oder über einen unterhalb des Seitenabzugs für die rohe Acrylsäure gelegenen Seitenabzug Acrylsäure-Oligomere enthaltende Schwersiederfraktion oder ein Gemisch aus solcher Acrylsäure-Oligomere enthaltender Sumpfflüssigkeit und Schwersiederfraktion aus der Kondensationskolonne entnimmt und als Quenchflüssigkeit 1 verwendet, den beim Abkühlen des Produktgasgemisches nicht verdampften Teil der Quenchflüssigkeit 1, gegebenenfalls über den Sumpf oder über die Schwersiederentnahme der Kondensationskolonne oder über beide sowie gegebenenfalls über einen Wärmetauscher, im Kreis führt und als Auslass einen Teil der Quenchflüssigkeit 1 aus diesem Kreislauf ausschleust und einem Spaltgefäß zuführt sowie in selbigem die im Auslass der Quenchflüssigkeit 1 enthaltenen Acrylsäure-Oligomere bei erhöhter Temperatur in Acrylsäure rückspaltet und dabei aus der Flüssigphase gasförmig entweichende, Acrylsäure enthaltende, Spaltgase in den Kreislauf der Quenchflüssigkeit 1 oder in die Kondensationskolonne oder in den Kreislauf der Quenchflüssigkeit 1 und in die Kondensationskolonne rückführt, wobei diese Rückführung gasförmig, oder kondensiert, oder teilkondensiert erfolgen kann.

Der Begriff rohe Acrylsäure bzw. Rohacrylsäure bringt dabei zum Ausdruck, dass es sich bei der über den Seitenabzug entnommenen Acrylsäure um kein reines Produkt, sondern um ein Gemisch handelt, das neben Acrylsäure (in der Regel ≥ 90, oder ≥ 95 % des Gesamtgewichtes) noch typische Nebenprodukte der Gasphasenoxidation wie z.B. Wasser, niedere Aldehyde (z.B. Furfurale, Acrolein, Benzaldehyd), niedere Carbonsäuren (z.B. Essigsäure, Propionsäure) enthält. Acrylsäure ist unter anderem durch heterogen katalysierte Gasphasen-Partialoxidation von C₃-Vorläufern der Acrylsäure (unter diesem Begriff werden solche chemischen Verbindungen zusammengefasst, die formal durch Reduktion von Acrylsäure erhältlich sind; bekannte C₃-Vorläufer von Acrylsäure sind z.B. Propan, Propen, Acrolein, Propionaldehyd und Propionsäure) mit molekularem Sauerstoff an im festen Aggregatzustand befindlichen Katalysatoren bei erhöhter Temperatur erhältlich. Dabei werden die genannten Ausgangsgase, in der Regel mit inerten Gasen wie z.B. Stickstoff, CO₂, gesättigten Kohlenwasserstoffen und/oder Wasserdampf verdünnt, im Gemisch mit molekularem Sauerstoff bei erhöhten Temperaturen sowie gegebenenfalls erhöhtem Druck über übergangsmetallische Mischoxidkatalysatoren geleitet und oxidativ in ein Acrylsäure sowie Nebenkomponenten wie z.B. Furfurale, Benzaldehyd und Maleinsäureanhydrid enthaltendes Produktgasgemisch umgewandelt, aus welchem die Acrylsäure abgetrennt werden muss.

Von Propionaldehyd und/oder Propionsäure ausgehend handelt es sich bei der heterogen katalysierten Gasphasen-Partialoxidation mit molekularem Sauerstoff wenigstens teilweise um eine oxidative Dehydrierung.

Aus der DE-A 199 24 533 ist ein wie eingangs beschriebenes Verfahren zur Herstellung von Acrylsäure bekannt, bei dem eine Grundabtrennung einer rohen Acrylsäure durch fraktionierende Kondensation des Produktgasgemisches der heterogen katalysierten Gasphasen-Partialoxidation vorgenommen wird. Ähnliche Verfahren der Grundabtrennung einer rohen Acrylsäure sind aus der DE-A 199 24 532, aus der WO 01/77056, aus der DE-A 10156016, aus der DE-A 10243625, aus der DE-A 10223058 und aus der DE-A 10235847 bekannt.

Die Rückspaltung der Acrylsäure-Oligomeren verfolgt dabei das Ziel, die Ausbeute an Wertprodukt zu erhöhen.

Ursächlich für die Entstehung von Acrylsäure-Oligomeren ist, dass in kondensierter Phase befindliche Acrylsäure durch reversible Michael-Addition an sich selbst sowie an das sich dabei bildende Acrylsäure-Dimere Acrylsäure-Oligomere (Michael-Addukte) bildet (der Begriff Acrylsäure-Oligomere meint in dieser Schrift stets die entsprechenden Michael-Addukte und nicht durch radikalische Polymerisation entstehende Acrylsäureoligomere).

Ein Beisein von Wasser, dem unvermeidbaren Nebenprodukt einer gasphasenkatalytisch oxidativen Herstellung von Acrylsäure sowie erhöhte Temperaturen fördern die Bildung von Acrylsäure-Oligomeren.

Da Acrylsäure-Oligomere eine höhere Siedetemperatur als Acrylsäure aufweisen, reichern sie sich sowohl im Rahmen einer destillativen Abtrennung von Acrylsäure als auch bei einer fraktionierenden Kondensation des Produktgasgemisches einer gasphasenkatalytisch oxidativen Acrylsäureerzeugung im Schwersiederbereich (z.B. in der Sumpfflüssigkeit) an.

Durch die Einwirkung erhöhter Temperatur unter gleichzeitiger Abtrennung der gebildeten Acrylsäure kann die Michael-Addition umgekehrt werden.

In den Beispielen der DE-A 199 24 533 sitzt dem Spaltgefäß(reaktor) stets ein Spritzschutz auf (z.B. eine kurze, mit Raschigringen gefüllte Kolonne), über den die Acrylsäure enthaltenden Spaltgase gasförmig und ohne Rücklauf abgetrennt und nach nach-folgend erfolgter Kondensation rückgeführt werden.

Nachteilig an einer solchen Verfahrensweise ist, dass die Reinheit der der Kondensationskolonne entnommenen rohen Acrylsäure nicht voll zu befriedigen vermag. Dies gilt insbesondere dann, wenn die rohe Acrylsäure kristallisativ, extraktiv und/oder rektifikativ weitergereinigt und dabei anfallende Mutterlauge, Sumpfflüssigkeit, Raffinat und/oder Kondensat in die Kondensationskolonne für die fraktionierende Kondensation des Produktgasgemisches der GasphasenPartialoxidation rückgeführt wird.

Die Aufgabe der vorliegenden Erfindung bestand daher darin, ein verbessertes Verfahren der Grundabtrennung von Acrylsäure aus dem Produktgasgemisch der heterogen katalysierten Gasphasen-Partialoxidation zur Verfügung zu stellen.

Demgemäß wurde ein Verfahren zur Herstellung von Acrylsäure, bei dem man durch heterogen katalysierte Gasphasen-Partialoxidation wenigstens eines C₃-Vorläufers der Acrylsäure mit molekularem Sauerstoff an im festen Aggregatzustand befindlichen Katalysatoren bei erhöhter Temperatur ein Acrylsäure enthaltendes Produktgasgemisch erzeugt, die Temperatur des heißen, Acrylsäure enthaltenden Produktgasgemisches durch direkte Kühlung mit einer Quenchflüssigkeit 1 zunächst verringert und anschließend das abgekühlte, gegebenenfalls Teile an verdampfter Quenchflüssigkeit 1 enthaltende, Produktgasgemisch in eine mit trennwirksamen Einbauten ausgerüstete Kondensationskolonne leitet, innerhalb der Kondensationskolonne in sich selbst aufsteigen lässt, dabei fraktionierend kondensiert sowie im Seitenabzug als Zielprodukt eine rohe Acrylsäure aus der Kondensationskolonne entnimmt und aus dem Sumpf der Kondensationskolonne Acrylsäure-Oligomere enthaltende Sumpfflüssigkeit oder über einen unterhalb des Seitenabzugs für die rohe Acrylsäure gelegenen Seitenabzug Acrylsäure-Oligomere enthaltende Schwersiederfraktion oder ein Gemisch aus solcher Acrylsäure-Oligomere enthaltender Sumpfflüssigkeit und Schwersiederfraktion aus der Kondensationskolonne entnimmt und als Quenchflüssigkeit 1 verwendet, den beim Abkühlen des Produktgasgemisches nicht verdampften Teil der Quenchflüssigkeit 1, gegebenenfalls über den Sumpf oder über die Schwersiederentnahme der Kondensationskolonne oder über beide sowie gegebenenfalls über einen Wärmetauscher, im Kreis führt und als Auslass einen Teil der Quenchflüssigkeit 1 aus diesem Kreislauf ausschleust und einem Spaltgefäß zuführt sowie in selbigem die im Auslass der Quenchflüssigkeit 1 enthaltenen Acrylsäure-Oligomere bei erhöhter Temperatur in Acrylsäure rückspaltet und dabei aus der Flüssigphase gasförmig entweichende, Acrylsäure enthaltende, Spaltgase in den Kreislauf der Quenchflüssigkeit 1 oder in die Kondensationskolonne oder in den Kreislauf der Quenchflüssigkeit 1 und in die Kondensationskolonne rückführt, wobei diese Rückführung gasförmig, oder kondensiert, oder teilkondensiert erfolgen kann, gefunden, das dadurch gekennzeichnet ist, dass die Spaltgase vor ihrer Rückführung einer Gegenstrom-Rektifikation unterworfen werden.

Der Vorteil des erfindungsgemäßen Verfahrens besteht insbesondere darin, dass bei gleicher Ausbeute an roher Acrylsäure der Gehalt der der Kondensationskolonne über Seitenabzug entnommenen rohen Acrylsäure an Nebenprodukten, die bei Normaldruck (1 bar) geringfügig höher wie Acrylsäure sieden (z.B. Benzaldehyd, Furfurale und Maleinsäureanhydrid), vermindert ist. Dem entspricht umgekehrt bei gleichem Nebenkomponentengehalt eine erhöhte Ausbeute an roher Acrylsäure. Dies ist vor allem dann von Bedeutung, wenn die rohe Acrylsäure kristallisativ, extraktiv und/oder rektifikativ weitergereinigt und dabei anfallende Mutterlauge, Sumpfflüssigkeit, Raffinat und/oder Kondensat in die Kondensationskolonne für die fraktionierende Kondensation des Produktgasgemisches der Gasphasen-Partialoxidation rückgeführt wird.

Insbesondere dann, wenn dabei das Verhältnis P_{F} : R_{F} des Gehaltes P_{F} an Furfuralen in der entnommenen rohen Acrylsäure, ausgedrückt als Gew.-% der in der rohen Acrylsäure enthaltenen Acrylsäure, und des Gehaltes R_{F} an Furfuralen im durch diese z.B. kristallisative Weiterreinigung erhaltenen Reinprodukt, ebenfalls ausgedrückt als Gew.-% der im Reinprodukt enthaltenen Acrylsäure, ≥ 300, bevorzugt ≥ 1000, besonders bevorzugt ≥ 2000, in der Regel aber ≤ 50000 beträgt.

Bei der erfindungsgemäß auf die Spaltgase anzuwendenden Gegenstrom-Rektifikation werden die Spaltgase und Rücklaufflüssigkeit im Gegenstrom zueinander durch eine Rektifikationskolonne geführt.

Als Rektifikationskolonne können dazu alle an sich bekannten Typen von Rektifikationskolonnen verwendet werden. Dies sind alle trennwirksame Einbauten enthaltenden Kolonnen, wobei als Einbauten z.B. Packungen, Füllkörper und/oder Böden in Betracht kommen. Prinzipiell können auch Kolonnen mit rotierenden Einsätzen, sogenannte Rotationskolonnen, verwendet werden, die die Rücklaufflüssigkeit in Tröpfchen versprühen.

Erfindungsgemäß bevorzugt wird als Rektifikationskolonne für die Spaltgase eine Rektifikationskolonne eingesetzt, die nur Böden und/oder Packungen enthält. Als Böden werden dabei mit Vorteil Dual-Flow-Böden verwendet und mit besonderem Vorteil enthält die Rektifikationskolonne ausschließlich Dual-Flow-Böden als trennwirksame Einbauten.

Unter Dual-Flow-Böden werden in dieser Schrift Platten mit einfachen Durchtrittstellen (Löcher, Schlitze) verstanden. Das in der Rektifikationskolonne aufsteigende Gas und die in der Rektifikationskolonne absteigende Rücklaufflüssigkeit treten entgegengesetzt strömend durch die gleichen Durchtrittstellen. Der Querschnitt der Durchtrittstellen wird in an sich bekannter Weise der Belastung der Rektifikationskolonne angepasst. Ist er zu klein, strömt das aufsteigende Spaltgas mit so hoher Geschwindigkeit durch die Durchtrittstellen, dass die in der Rektifikationskolonne absteigende Rücklaufflüssigkeit im wesentlichen ohne Trennwirkung mitgerissen wird. Ist der Querschnitt der Durchtrittstellen zu groß, bewegen sich aufsteigendes Spaltgas und absteigender Rücklauf im wesentlichen ohne Austausch aneinander vorbei und der Boden läuft Gefahr trocken zu laufen. Üblicherweise weisen Dual-Flow-Böden kein Ablaufrohr auf, das sie mit dem nächsten Boden verbindet. Selbstredend kann jeder Dual-Flow-Boden mit den Wänden der Rektifikationskolonne bündig abschließen. Er kann aber auch über Stege mit diesen verbunden sein. Mit abnehmender Belastung der Rektifikationskolonne laufen Dual-Flow-Böden im Unterschied zu hydraulisch abgedichteten Querstromböden trocken.

Erfindungsgemäß zweckmäßig kann die erfindungsgemäß für die Spaltgase verwendbare Dual-Flow-Boden-Rektifikationskolonne bis zu 60 Dual-Flow-Böden enthalten. In der Regel wird sie 30 bis 50 Dual-Flow-Böden enthalten. Mit Vorteil weisen diese ein Öffnungsverhältnis (das Verhältnis D:U, gebildet aus dem Flächenanteil des Bodens, der für das Spaltgas durchlässig ist (D) und der Gesamtfläche des Bodens (U)) von 10 bis 20 %, bevorzugt von 10 bis 15 % auf.

Die Durchtrittstellen der Dual-Flow-Böden sind bevorzugt kreisrunde Löcher mit einem innerhalb des Bodens einheitlichen Kreisdurchmesser. Letzterer beträgt zweckmäßig 10 bis 30 mm. Im oberen Teil der Kolonne beträgt er mit Vorteil 10 bis 20 mm, bzw. 10 bis 15 mm, und im unteren Teil der Kolonne beträgt er mit Vorteil 20 bis 30 mm. Ferner sind die kreisrunden Löcher über den individuellen Dual-Flow-Böden bevorzugt in strenger Dreiecksteilung gleichmäßig angeordnet (vgl. DE-A 10230219). Ferner zeigt der Stanzgrat der in den erfindungsgemäß mitzuverwendenden Dual-Flow-Böden herausgestanzten Durchtrittsöffnungen in der erfindungsgemäß zu verwendenden Rektifikationskolonne bevorzugt nach unten. Üblicherweise sind die Dual-Flow-Böden äquidistant in der Rektifikationskolonne angeordnet. In typischer Weise liegt der Bodenabstand bei 300 mm bis 500 mm. Erfindungsgemäß günstig ist auch ein Bodenabstand von 400 mm. In einfacher Weise lässt sich die erfindungsgemäß zu integrierende thermische Rückspaltung in einem Zwangsumlaufentspannungsverdampfer durchführen. Anstelle eines Zwangsumlaufentspannungsverdampfers kann aber auch z.B. ein Robertverdampfer oder ein Naturumlaufverdampfer eingesetzt werden. Spaltgefäß(reaktor) und Rektifikationskolonne können sowohl räumlich getrennt sein, als auch nahtlos ineinander übergehen. In beiden Fällen führt man beim erfindungsgemäßen Verfahren den aus dem Kreislauf ausgeschleusten Teil der Quenchflüssigkeit 1 in zweckmäßiger Weise nicht unmittelbar in das Spaltgefäß, sondern über die diesem aufgesetzte Rektifikationskolonne, vorzugsweise über deren unteren Teil (z.B. letztes Drittel). In zweckmäßiger Weise erfolgt die Zufuhr auf den vierten bis zehnten Dual-Flow-Boden der Rektifikationskolonne (von unten gerechnet). Die Rückspalttemperatur liegt beim erfindungsgemäßen Verfahren in zweckmäßiger Weise bei 160 bis 190°C, bevorzugt bei 165 bis 175°C. Der für die Rückspaltung gewählte Arbeitsdruck wird vorteilhaft etwas oberhalb (z.B. 100 mbar oberhalb) des Druckes im Quenchkreis 1 liegen. Ein typischer Bereich für für die Rückspaltung geeignete Arbeitsdrucke beträgt 1 bar bis 2 bar.

Es kann im übrigen erfindungsgemäß zweckmäßig sein, die Rückspaltung unter Zusatz eines anorganischem Salzes, dessen Zusatz zu einer wässrigen Lösung einer starken Brönsted-Säure den pH-Wert der wässrigen Lösung ins alkalische verschiebt, als Katalysator vorzunehmen, wie es z.B. die DE-C 2 407 236 empfiehlt. Bezogen auf die der Rückspaltung zu unterwerfende Menge an ausgeschleuster Quenchflüssigkeit 1 wird die zuzusetzende Menge an basischem Rückspaltkatalysator in der Regel 0,1 bis 5 Gew.-% betragen. Beispiele für erfindungsgemäß geeignete Rückspaltkatalysatoren sind KOH, K₂CO₃, KHCO₃, NaOH, Na₂CO₃, NaHCO₃, LiOH, Li₂CO₃ und CaCO₃. D.h., geeignete Rückspaltkatalysatoren sind insbesondere die Alkali- und/oder Erdalkalisalze von schwachen anorganischen oder organischen Brönstedsäuren wie z.B. Phosphorsäure, Borsäure, Kohlensäure, Ameisensäure oder Essigsäure. Mit anderen Werten eignen sich somit als Rückspaltkatalysatoren vor allem Alkali- und/oder Erdalkaliphosphate, -borate, -carbonate, -hydrogencarbonate, - formiate und -acetate.

Vorzugsweise wird man die Rückspaltkatalysatoren so wählen, dass sie unter den Rückspaltbedingungen in der ausgeschleusten Quenchflüssigkeit 1 löslich sind. Gemäß US-A 4293347 wirkt sich auch ein Beisein von Dialkylphthalaten vorteilhaft auf die relevante Rückspaltung aus.

Wird die Rückspaltung kontinuierlich durchgeführt (das erfindungsgemäße Verfahren wird vorzugsweise kontinuierlich durchgeführt), so sollte die Verweilzeit im Rückspaltreaktor 0,5 h bis 4 h betragen. Wie in der US-A 5733075 sowie in der DE-A 4101879 beschrieben, lässt sich die Rückspaltung der Acrylsäure-Oligomeren prinzipiell auch ohne Zusatz von Spaltkatalysatoren durchführen. Diese Verfahrensweise ist erfindungsgemäß bevorzugt. Es können aber auch saure Spaltkatalysatoren mitverwendet werden. Als solche kommen Dodecylbenzolsulfonsäure, p-Toluolsulfonsäure, Schwefelsäure oder die festen sauren Katalysatoren der JP-A 178949 in Betracht.

Erfindungsgemäß vorteilhaft ist es, wenn der Rückspaltreaktor (das Spaltgefäß) und die Rektifikationskolonne (vorteilhaft ist auch schon eine alleinige Durchströmung der Rektifikationskolonne) für das Spaltgas von einem Sauerstoff enthaltenden Gas durchströmt wird. Als solches kommt z.B. Luft, an Sauerstoff entreicherte Luft und/oder Kreisgas in Betracht. Unter Kreisgas wird das Gas verstanden, das verbleibt, wenn man aus dem Produktgasgemisch der heterogen katalysierten Gasphasen-Partialoxidation in der Kondensationskolonne die einfach kondensierbaren (in erster Linie schwerer als Wasser flüchtige und Wasser) Bestandteile weitgehend auskondensiert. Es verlässt die Kondensationskolonne in der Regel an deren Kopf und kann z.B. die nachfolgende, für das erfindungsgemäße Verfahren im Fall einer von Propylen ausgehenden zweistufigen Gasphasen-Partialoxidation typische, Zusammensetzung aufweisen:
0,2651 Gew.-% Acrylsäure,
0,0989 Gew.-% Essigsäure,
2,9333 Gew.-% Wasser,
0,0059 Gew.-% Ameisensäure,
0,1720 Gew.-% Acrolein,
0,0002 Gew.-% Propionsäure,
0,0002 Gew.-% Furfurale,
0,0013 Gew.-% Allylformiat,
4,7235 Gew.-% Sauerstoff,
2,1171 Gew.-% CO₂,
0,6921 Gew.-% CO,
0,6466 Gew.-% Propan,
0,3161 Gew.-% Propylen und
88,0277 Gew.-% Stickstoff.

In anwendungstechnisch zweckmäßiger Weise führt man das Sauerstoff enthaltende Gas (nachfolgend "Unterstützgas" genannt) unmittelbar in den Spaltreaktor, von wo es zusammen mit den Spaltgasen automatisch der Rektifikationskolonne zugeführt wird. Es könnte aber auch unmittelbar in die Rektifikationskolonne gegeben werden (zweckmäßigerweise unterhalb des ersten Bodens (von unten)). Die Mitverwendung des Sauerstoff enthaltenden Gases wirkt einerseits polymerisationsinhibierend und unterstützt andererseits die Förderung der Spaltgase in die Rektifikationskolonne. Zudem reduziert es den Spaltgaspartialdruck. Anstelle eines Sauerstoff enthaltenden Gases könnte auch ein NO-haltiges Gas verwendet werden. Sowohl im Fall des Sauerstoff als auch im Fall des NO handelt es sich bei den übrigen Gasbestandteilen um Inertgas.

Die Rektifikationskolonne ist (wie auch die Kondensationskolonne) zweckmäßig gegen die Umgebung isoliert. Die Erzeugung der Rücklaufflüssigkeit kann durch direkte und/oder indirekte Kühlung erfolgen. Erfindungsgemäß vorteilhaft wird die Methode der direkten Kühlung angewendet. Dazu werden in einfachster Weise die Spaltgase (bzw. deren Gemisch mit Unterstützgas), die die Rektifikationskolonne an deren Kopf verlassen, einer Quenchvorrichtung 2 zugeführt. Als Quenchvorrichtung 2 können alle im Stand der Technik für diesen Zweck bekannten Vorrichtungen (z.B. Sprühwäscher, Venturiwäscher, Blasensäulen oder sonstige Apparate mit berieselten Oberflächen) eingesetzt werden, wobei vorzugsweise Venturi-Wäscher oder Sprühkühler verwendet werden. Bevorzugt wird eine Gleichstromvorrichtung (z.B. eine mit Prallplattendüse) verwendet. Zur indirekten Kühlung der Quenchflüssigkeit 2 wird diese üblicherweise über einen Wärmeüberträger bzw. Wärmetauscher geführt. Diesbezüglich eignen sich alle gängigen Wärmeüberträger oder Wärmetauscher. Als bevorzugt seien Rohrbündelwärmetauscher, Plattenwärmetauscher und Luftkühler genannt. Geeignete Kühlmedien sind Luft beim entsprechenden Luftkühler und Kühlflüssigkeiten, insbesondere Wasser, bei den anderen Kühlvorrichtungen.

Als Quenchflüssigkeit 2 wird anwendungstechnisch zweckmäßig eine Teilmenge des beim Quenchen 2 gebildeten Kondensats verwendet. Die andere Teilmenge des beim Quenchen 2 gebildeten Kondensats wird am Kopf der Rektifikationskolonne als Rücklaufflüssigkeit in selbige rückgeführt. In typischer Weise beträgt die Temperatur der Quenchflüssigkeit 2 unmittelbar vorab ihrer Verwendung zum Quenchen 2 etwa 30°C, wohingegen die Rücklaufflüssigkeit in typischer Weise mit etwa 60°C in die Rektifikationskolonne rückgeführt wird. Die Spaltgase selbst bzw. deren Gemisch mit Unterstützgas verlassen die Rektifikationskolonne an deren Kopf in der Regel mit einer Temperatur von 90 bis 100°C. Selbstredend kann der Spaltgasquench (der Quenchkreis 2) auch in die Rektifikationskolonne integriert werden.

Das Massenverhältnis von in die Rektifikationskolonne rückgeführter Rücklaufflüssigkeit zu der Spaltvorrichtung, zugeführter ausgeschleuster Quenchflüssigkeit 1 beträgt typisch ≥ 2. Häufig beträgt es 2 bis 10 und vorzugsweise 4 bis 8.

Selbstverständlich muss die Spaltgas-Rektifikationskolonne polymerisationsinhibiert betrieben werden. Als Polymerisationsinhibitoren können für diesen Zweck prinzipiell alle im Stand der Technik bekannten Polymerisationsinhibitoren eingesetzt werden. Beispielhaft genannt seien als solche Phenothiazin (PTZ) und p-Methoxyphenol (MEHQ). Häufig werden die letzten beiden kombiniert angewendet. Zweckmäßigerweise werden diese dazu in einer reinen Acrylsäure gelöst zugesetzt.

Insbesonders eleganter Weise lässt sich die Polymerisationsinhibierung des Quenchkreises 2 und der Rektifikationskolonne für die Spaltgase jedoch dadurch bewerkstelligen, dass man dem Quenchkreis 2 eine Teilmenge der ausgeschleusten, polymerisationsinhibierten, d.h. Polymerisationsinhibitoren enthaltenden, Quenchflüssigkeit 1 zusetzt. In typischer Weise enthält die Quenchflüssigkeit 2 so z.B. 0,01 bis 0,02 Gew.-% MEHQ und 0,01 bis 0,02 Gew.-% PTZ.

Das den Quenchkreis 2 gekühlt verlassende Spaltgas bzw. Gemisch aus Spaltgas und Unterstützgas kann erfindungsgemäß sowohl in den Quenchkreis 1 und/oder in die Kondensationskolonne rückgeführt werden. Dies kann sowohl flüssig als auch gasförmig erfolgen. Erfindungsgemäß bevorzugt erfolgt diese Rückführung gasförmig. Mit Vorteil wird in den Sumpfraum der Kondensationskolonne rückgeführt. Dabei kann diese Rückführung sowohl in die Sumpfflüssigkeit getaucht, als auch oberhalb des Flüssigkeitsspiegels der Sumpfflüssigkeit und unterhalb des ersten Bodens der Kondensationskolonne erfolgen. Anwendungstechnisch zweckmäßig enthält der Sumpfraum der Kondensationskolonne einen Tropfenabscheider (z.B. einen Zentrifugaltropfenabscheider), um ein Mitreissen von Sumpfflüssigkeitströpfchen durch aufsteigendes Gas zu unterdrücken.

Der im Spaltreaktor verbleibende schwerflüchtige Rückstand wird beim erfindungsgemäßen Verfahren regelmäßig seiner Entsorgung, z.B. seiner Verbrennung, zugeführt. Durch Zusatz eines organischen Lösungsmittels, z.B. von Methanol, wird der schwerflüchtige Spaltrückstand bei Bedarf fluid gehalten. Anstelle von Methanol können auch andere hydrophile organische Flüssigkeiten wie z.B. Ethanol oder organische Säuren (z.B. Ethylhexansäure, Propionsäure) oder Rückstände aus der Herstellung dieser Säuren verwendet werden. An dieser Stelle sei noch festgehalten, dass der im erfindungsgemäßen Verfahren zu verwendenden Quenchflüssigkeit 1 bei Bedarf eine höher als Acrylsäure siedende inerte organische Flüssigkeit zugesetzt werden kann, die die Quenchflüssigkeit 1 fluid hält. Als solche hochsiedenden inerten organischen Flüssigkeiten kommen insbesondere alle diejenigen in Betracht, die in der DE-A 2136396 und in der DE-A 4308087 empfohlen werden.

Dies sind im wesentlichen Flüssigkeiten, deren Siedepunkt bei Normaldruck oberhalb von 160°C liegt. Beispielhaft genannt seien Ethylhexansäure, N-Methylpyrrolidon, Mittelölfraktionen aus der Paraffindestillation, Diphenylether, Diphenyl oder Mischungen der vorgenannten Flüssigkeiten wie z.B. ein Gemisch aus 70 bis 75 Gew.-% Diphenylether und 25 bis 30 Gew.-% Diphenyl. Günstig sind die Verwendung eines Gemisches bestehend aus einer Mischung von 70 bis 75 Gew.-% Diphenylether und 25 bis 30 Gew.-% Diphenyl, sowie, bezogen auf diese Mischung, 0,1 bis 25 Gew.-% o-Dimethylphthalat. Im vorgenannten Fall wird bei der Rückspaltung wenigstens eine Teilmenge der mitverwendeten inerten organischen Flüssigkeit mitverdampfen. Verbleibt eine Teilmenge der organischen Flüssigkeit im Spaltrückstand, kann selbiger einer Aufarbeitung zugeführt werden, in der das mitverwendete Lösungsmittel, z.B. destillativ, abgetrennt und in den Quenchkreis 1 rückgeführt wird. Die verbleibenden Schwersieder werden entsorgt.

In typischer Weise ist das Acrylsäure enthaltende Produktgasgemisch einer heterogen katalysierten Gasphasen-Partialoxidation von C₃-Vorläufern der Acrylsäure mit molekularem Sauerstoff an in festen Aggregatzustand befindlichen Katalysatoren wie folgt zusammengesetzt (insbesondere dann, wenn als C₃-Vorläufer Propylen verwendet wird):
1 bis 30 Gew.-% Acrylsäure,
0,05 bis 10 Gew.-% molekularer Sauerstoff,
1 bis 30 Gew.-% Wasser,
> 0 bis 5 Gew.-% Essigsäure,
> 0 bis 3 Gew.-% Propionsäure,
> 0 bis 1 Gew.-% Maleinsäure und/oder Maleinsäure-Anhydrid,
0 bis 2 Gew.-% Acrolein,
0 bis 1 Gew.-% Formaldehyd,
> 0 bis 1 Gew.-% Furfurale,
> 0 bis 0,5 Gew.-% Benzaldehyd,
0 bis 1 Gew.-% Propylen, und als Restmenge inerte Gase
wie z.B. Stickstoff, Kohlenmonoxid, Kohlendioxid, Methan und/oder Propan.

Üblicherweise enthält das Produktgasgemisch, bezogen auf enthaltene Acrylsäure, ≥ 0,005 mol-%, häufig ≥ 0,03 mol-%, an Furfuralen. In der Regel beträgt der so bezogene Furfuralgehalt jedoch ≤ 3 mol.-%.

Die Gasphasen-Partialoxidation selbst kann wie im Stand der Technik beschrieben durchgeführt werden. Ausgehend von Propylen kann die Gasphasen-Partialoxidation z.B. in zwei aufeinanderfolgenden Oxidationsstufen durchgeführt werden, wie sie in der EP-A 700714 und in der EP-A 700893 beschrieben sind. Selbstverständlich können aber auch die in der DE-A 19740253 sowie in der DE-A 19740252 zitierten Gasphasen-Partialoxidationen zur Anwendung kommen.

Im Sinne einer geringen Menge gebildeter Nebenkomponenten wird die Propylen-Gasphasen-Parialoxidation bevorzugt wie in der DE-A 10148566 beschrieben durchgeführt. Als Propylenquelle kann dazu Polymer grade Propylen oder Chemical grade Propylen gemäß der DE-A 10232748 verwendet.werden. Handelt es sich bei dem verwendeten C₃-Vorläufer um Propan, kann die Partialoxidation wie in der DE-A 10245585 beschrieben durchgeführt werden.

Häufig beträgt die Temperatur des die Gasphasen-Partialoxidation verlassenden Produktgasgemisches 150 bis 350°C, vielfach 200 bis 300°C, manchmal bis zu 500°C.

In einer Quenchvorrichtung 1 wird das heiße Produktgasgemisch erfindungsgemäß durch direkte Kühlung in der Regel auf eine Temperatur von 100 bis 180°C abgekühlt, bevor es, anwendungstechnisch vorteilhaft gemeinsam mit der verwendeten Quenchflüssigkeit 1, zum Zweck der fraktionierenden Kondensation bevorzugt in den unteren Abschnitt (bevorzugt den untersten, z.B. den Sumpfraum) einer trennwirksamen Einbauten enthaltenden Kondensationskolonne geleitet wird.

Als Kondensationskolonneneinbauten kommen prinzipiell alle gängigen Einbauten in Betracht, insbesondere Böden, Packungen und/oder Füllkörper. Von den Böden sind Glockenböden, Siebböden, Ventilböden und/oder Dual-Flow-Böden bevorzugt. In typischer Weise beträgt bei einer Bodenkolonne die Gesamtzahl an Trennböden 20 bis 100, häufig 20 bis 80 und bevorzugt 50 bis 80.

Erfindungsgemäß bevorzugt ist die Kondensationskolonne eine solche, die von unten nach oben, zunächst Dual-Flow-Böden und im Anschluss daran hydraulisch abgedichtete Querstromböden (z.B. Thormann®-Böden) als trennwirksame Einbauten enthält, wie es die DE-A 19924532 und die DE-A 10243625 empfehlen. Die Anzahl der Dual-Flow-Böden kann dabei 5 bis 60, häufig 25 bis 45, und die Anzahl der hydraulisch abgedichteten Querstromböden ebenfalls 5 bis 60, häufig 30 bis 50 betragen. Wird der Sauerwasserquench (Quench 3) in die Kondensationskolonne integriert, so kommen für diesen Bereich der Kondensationskolonne (Acrylsäuregehalt der Rücklaufflüssigkeit von unten nach oben betrachtet in der Regel ≤10 Gew.-%) als trennwirksame Einbauten bevorzugt Ventilböden in Betracht, wie es die DE-A 19924532 und die DE-A 10243625 beschreiben. Prinzipiell könnten aber auch andere gängige trennwirksame Einbauten verwendet werden.

Als Quenchvorrichtung 1 können alle im Stand der Technik für diesen Zweck bekannten Vorrichtungen (z.B. Sprühwäscher, Venturiwäscher, Blasensäulen oder sonstige Apparate mit berieselten Oberflächen) eingesetzt werden, wobei vorzugsweise Venturi-Wäscher oder Sprühkühler verwendet werden.

Zur indirekten Kühlung oder Erwärmung der Quenchflüssigkeit 1 wird diese, insbesondere beim Anfahren, bevorzugt über einen Wärmeüberträger bzw. Wärmetauscher geführt. Diesbezüglich eignen sich alle gängigen Wärmeüberträger oder Wärmetauscher. Als bevorzugt seien Rohrbündelwärmetauscher, Plattenwärmetauscher und Luftkühler genannt. Geeignete Kühlmedien sind Luft beim entsprechenden Luftkühler und Kühlflüssigkeiten, insbesondere Wasser, bei den anderen Kühlvorrichtungen.

Als Quenchflüssigkeit 1 kann erfindungsgemäß z.B. aus dem Sumpf der Kondensationskolonne Acrylsäure-Oligomere enthaltende Sumpfflüssigkeit, oder über einen in Sumpfnähe gelegenen Seitenabzug Acrylsäure-Oligomere enthaltende Schwersiederfraktion oder, erfindungsgemäß bevorzugt, ein Gemisch aus solcher Acrylsäure-Oligomere enthaltender Sumpfflüssigkeit und Schwersiederfraktion verwendet werden. Vorzugsweise wird nur der aus dem Sumpf der Kondensationskolonne entnommene Anteil der Quenchflüssigkeit 1 über den oben genannten Wärmetauscher geführt. Die Temperatur der Quenchflüssigkeit 1 beträgt beim Eintritt in die Quenchvorrichtung 1 erfindungsgemäß zweckmäßig in der Regel 90°C bis 120°C.

Die Einleitstelle für das gequenchte Produktgasgemisch der katalytischen Gasphasen-Partialoxidation, erfindungsgemäß bevorzugt im Gemisch mit der zur direkten Kühlung verwendeten Quenchflüssigkeit 1, in die Kondensationskolonne befindet sich vorteilhaft im Sumpfraum dieser Kolonne, der mit Vorteil einen Zentrifugaltropfenabscheider integriert enthält und in der Regel durch einen ersten Kaminboden von der untersten trennwirksamen Einbaute getrennt ist. In der besonders bevorzugten Ausführungsvariante der Kondensationskolonne und des erfindungsgemäßen Verfahrens, die im folgenden ausschließlich beschrieben werden, handelt es sich bei dieser um den ersten Dual-Flow-Boden einer ersten Serie von zweckmäßig äquidistant angeordneten, Dual-Flow-Böden. Der Kaminboden fungiert gleichzeitig als Sammelboden, von dem kontinuierlich Kondensat (Acrylsäure-Oligomere enthaltende Schwersiederfraktion) entnommen und als Teil der Quenchflüssigkeit 1 in die Quenchvorrichtung 1 geführt wird. Die erste Serie von Dual-Flow-Böden wird von einem zweiten Kaminboden (Fangboden) abgeschlossen. Von diesem zweiten Fangboden wird im Seitenabzug als Mittelsiederfraktion rohe Acrylsäure kontinuierlich entnommen, die im Normalfall eine Reinheit von ≥ 90 bzw. ≥ 95 Gew.-% aufweist.

Zweckmäßigerweise wird man diese rohe Acrylsäure weiteren destillativen (rektifikativen) und/oder kristallisativen Weiterreinigungsstufen zuführen und wenigstens einen Teil der im Rahmen dieser Destillation (Rektifikation) und/oder Kristallisation anfallenden Sumpfflüssigkeiten und/oder Mutterlaugen unterhalb des zweiten, aber oberhalb des ersten Fangbodens in die Kondensationskolonne rückführen. Vorzugsweise erfolgt diese Rückführung wärmeintegriert. D.h., die kalte rückzuführende Mutterlauge wird über eine oder mehrere hintereinander angeordnete indirekte Wärmeaustauschstufen verwendet, um die der Kondensationskolonne entnommene, kristallisativ weiterzureinigende, rohe Acrylsäure abzukühlen. Gleichzeitig wird dadurch eine Erwärmung der Mutterlauge bewirkt.

In zweckmäßiger Weise wird man die als Mittelsiederfraktion entnommene rohe Acrylsäure zum Zweck der Weiterreinigung einer Kristallisation zuführen. Das zu verwendende Kristallisationsverfahren unterliegt prinzipiell keiner Beschränkung. Die Kristallisation kann kontinuierlich oder diskontinuierlich, einstufig oder mehrstufig bis zu beliebigen Reinheitsgraden durchgeführt werden.

Bei Bedarf kann der kristallisativ zu reinigenden rohen Acrylsäure vorab der Kristallisation Wasser zugesetzt werden (in der Regel enthält diese dann, bezogen auf die enthaltene Menge an Acrylsäure, bis zu 10 Gew.-%, meist bis zu 5 Gew.-% an Wasser). Bei erhöhten Aldehyd- oder sonstigen Nebenkomponentengehalten kann eine Wasserzugabe unterbleiben, da die Aldehyde in diesem Fall die Funktion des Wasser zu übernehmen vermögen.

Es überrascht, dass eine veresterungsgerechte (z.B. für die Herstellung von n-Butylacrylat, 2-Ethylhexylacrylat, Methylacrylat und Ethylacrylat) Acrylsäure (Reinheit ≥ 98 Gew.-%) bereits durch eine einzige Kristallisationsstufe erzielt werden kann. Zweckmäßigerweise wird diese Kristallisationsstufe als Suspensionskristallisation ausgeführt, wie es in Spalte 10 der DE-A 19924532 bzw. in Beispiel 1 der DE-A 10223058 beschrieben ist. Die bei der Suspensionskristallisation entstehenden Acrylsäure-Kristalle haben eine würfel- bis quaderförmige Erscheinungsform. Das Länge (L) zu Dicke (D) Verhältnis liegt dabei üblicherweise im Bereich von L:D = 1:1 bis L:D = 6:1, bevorzugt im Bereich 1:1 bis 4:1, und besonders bevorzugt im Bereich 1,5:1 bis 3,5:1. Die Dicke D der Kristalle liegt üblicherweise im Bereich von 20 bis 600 µm, oft bei 50 bis 300 µm. Die Länge L der Kristalle liegt üblicherweise im Bereich von 50 bis 1500 µm, oft bei 200 bis 800 µm. Die Abtrennung des Suspensionskristallisats von der verbliebenen Mutterlauge kann im Fall von veresterungsgerechter Acrylsäure auf einer Zentrifuge (z.B. einer 2- oder 3-stufigen Schubzentrifuge) erfolgen, wobei die abgetrennten Kristalle in vorteilhafter Weise auf der Zentrifuge mittels aufgeschmolzenem Reinkristallisat gewaschen werden. Trennt man das Suspensionskristallisat von der verbliebenen Mutterlauge mittels einer Schmelz-Waschkolonne (z.B. einer solchen gemäß der WO 01/77056, oder der DE-A 10156016, oder der DE-A 10223058), kann mittels einer einzigen Kristallisationsstufe sogar superabsorbergerechte Acrylsäure (Reinheit ≥ 99,7 Gew.-%), d.h., Acrylsäure, die sich zur Herstellung von Wasser superabsorbierenden oder sonstigen Polyacrylaten eignet, erzielt werden. In diesem Fall führt man in zweckmäßiger Weise die Gesamtmenge der abgetrennten Mutterlauge in die erfindungsgemäße Trennkolonne zurück.

Die Kristallisation kann aber auch als fraktionierte Fallfilmkristallisation durchgeführt werden, wie sie die EP-A 616998 empfiehlt. Diese kann z.B. drei oder mehr (z.B. 3 bis 4) Reinigungsstufen umfassen (diesbezüglich geeignete Fallfilmkristallisatoren können z.B. 1000 bis 1400 Kristallisationsrohre einer Länge von 10 bis 15 m und eines Außendurchmessers von 50 bis 100 mm enthalten). Die in einer höheren Reinigungsstufe abgetrennte Mutterlauge kann in eine der vorgehenden Reinigungsstufen rückgeführt werden. Die in der ersten Reinigungsstufe abgetrennte Mutterlauge wird vorteilhaft vollständig in die Kondensationskolonne rückgeführt. Alternativ zur Rückführung in eine der vorhergehenden Reinigungsstufen können die Mutterlaugen der einzelnen Reinigungsstufen auch in ihrer Gesamtmenge in die Kondensationskolonne rückgeführt werden. Das Reinprodukt der vorletzten Reinigungsstufe kann vollständig oder nur teilweise der letzten Reinigungsstufe zugeführt werden. Erfolgt nur eine Teilzuführung wird man die verbleibende Restmenge in der Regel mit dem Reinprodukt der letzten Reinigungsstufe zum dann verbrauchsgerechten Endprodukt abmischen.

Erfindungsgemäß zweckmäßig wird man eine Teilmenge der dem zweiten Fangboden entnommenen rohen Acrylsäure dem unterhalb dieses Fangbodens befindlichen Dual-Flow-Boden zuführen. Diesem Boden wird man in der Regel auch gegebenenfalls in die Kondensationskolonne rückzuführende Mutterlauge zuführen. Vorab der Zufuhr wird man die Mutterlauge in der Regel, wie bereits beschrieben, wärmeintegriert auf eine Temperatur erwärmen, die in etwa der Entnahmetemperatur der rohen Acrylsäure entspricht.

Eine andere Teilmenge der dem zweiten Fangboden entnommenen rohen Acrylsäure wird man durch indirekten Wärmeaustausch um 10 bis 15°C erwärmen und oberhalb der Entnahmestelle, bevorzugt unmittelbar unterhalb des ersten nachfolgenden Dual-Flow-Bodens, in die Kondensationskolonne rückführen. Diese Maßnahme wirkt sich günstig auf den Essigsäuregehalt der entnommenen rohen Acrylsäure aus.

Oberhalb des zweiten Fangbodens schließt sich zunächst eine zweite Serie von, zweckmäßigerweise äquidistanten, Dual-Flow-Böden an, die dann von hydraulisch abgedichteten Querstrom-Stoffaustauschböden (z.B. Thormann-Böden oder modifizierte Thormann-Böden gemäß DE-A 10243625), die zweckmäßigerweise gleichfalls äquidistant angeordnet sind, abgelöst werden. Der oberste Dual-Flow-Boden ist häufig als Verteilerboden ausgerüstet. D.h., er weist z.B. Überlaufrinnen mit gezacktem Überlauf auf.

Die Querstrom-Stoffaustauschböden werden mit einem dritten Kaminboden (Fangboden) abgeschlossen.

Oberhalb des dritten Fangbodens befinden sich, vorzugsweise zweiflutige, Ventilböden. Das Prinzip von Ventilböden sowie erfindungsgemäß verwendbare Ventilböden finden sich z.B. in Technische Fortschrittsberichte, Band 61, Grundlagen der Dimensionierung von Kolonnenböden, S. 96 bis 138. Sie sind im wesentlichen dadurch charakterisiert, dass sie dem durchströmenden Dampf über einen weiten Belastungsbereich eine der jeweiligen Belastung entsprechende Durchstromöffnung zur Verfügung stellen. Erfindungsgemäß bevorzugt werden Ballastböden verwendet. D.h., in den Öffnungen des Bodens befinden sich Käfige mit durch Gewichte verschlossenen Öffnungen. Erfindungsgemäß besonders bevorzugt sind W12-Ventile der Fa. Stahl, DE, Viernheim. Im Ventilbodenraum kondensiert im wesentlichen Wasser sowie schwerer als Wasser flüchtige Bestandteile. Das dabei gewonnene Kondensat wird als Sauerwasser bezeichnet.

Vom dritten Fangboden wird kontinuierlich Sauerwasser entnommen. Ein Teil des entnommenen Wassers wird auf den obersten der Querstrom-Stoffaustauschböden in die Kondensationskolonne rückgeführt. Ein anderer Teil des entnommenen Sauerwassers wird durch indirekten Wärmetausch abgekühlt und, in zweckmäßiger Weise gesplittet, ebenfalls in die Kondensationskolonne rückgeführt. Eine Teilmenge wird dabei auf den obersten Ventilboden (mit einer Temperatur von 15 bis 25, bevorzugt 20 bis 25°C) und die andere Teilmenge auf einen zwischen dem dritten Fangboden und dem obersten Ventilboden etwa mittig gelegenen Ventilboden in die erfindungsgemäße Trennkolonne rückgeführt (mit einer Temperatur von 20 bis 35, bevorzugt 25 bis 30°C).

Ein Teil der Abkühlung wird dadurch bewirkt, dass das Sauerwasser über den Verdampfer des C₃-Vorläufers (z.B. den Propylenverdampfer) geführt wird, um flüssig gelagerten C₃-Vorläufer, z.B. Propylen, für die heterogen katalysierte Gasphasenoxidation in die Gasphase zu überführen.

Leichter als Wasser flüchtige Bestandteile werden am Kopf der erfindungsgemäßen Trennkolonne gasförmig abgezogen und im Normalfall wenigstens teilweise als Verdünnungsgas in die Gasphasenoxidation rückgeführt. Um im Kreisgasverdichter Kondensation zu vermeiden, wird das Abgas zuvor durch indirekten Wärmeaustausch überhitzt. Der nicht im Kreis geführte Teil des Abgases wird normalerweise der Verbrennung zugeführt. Ein Teil des Kreisgases wird, wie bereits beschrieben, in vorteilhafter Weise als Unterstützgas bei der Spaltung der Acrylsäure-Oligomere mitverwendet.

Zum Zweck der Polymerisationsinhibierung wird dem obersten der hydraulisch abgedichteten Querstrom-Stoffaustauschböden eine Lösung von p-Methoxyphenol (= MEHQ) in Acrylsäure und gegebenenfalls zusätzlich eine Lösung von Phenothiazin in Acrylsäure zugeführt. Als Acrylsäure wird dabei vorzugsweise eine reine Acrylsäure verwendet, wie sie bei der Weiterreinigung der entnommenen rohen Acrylsäure erzeugt wird. Beispielsweise kann die im Rahmen der kristallisativen Weiterreinigung erzeugte Reinacrylsäure (Reinprodukt) verwendet werden. Diese Lösung wird zweckmäßig auch zur Reinproduktstabilisierung verwendet.

Zusätzlich wird etwa in der Mitte des Kolonnenabschnitts mit den hydraulisch abgedichteten Querstrom-Stoffaustauschböden eine Lösung von Phenothiazin (= PTZ) in Reinprodukt zugeführt.

Prinzipiell kann die Sauerwasserbildung auch außerhalb der Kondensationskolonne praktiziert werden. In diesem Fall wird man aus dem dann am Kopf der Kondensationskolonne entweichenden Leichtsiedergasstrom in zweckmäßiger Weise durch direkte Kühlung in einem an Einbauten freien oder Einbauten enthaltenden Raum mittels einer Quenchflüssigkeit 3 im wesentlichen Wasser auskondensieren. Das dabei gewonnene Kondensat ist wiederum das Sauerwasser. Einen Teil des Sauerwassers wird man dann in sinnvoller Weise zur Erhöhung der Trennleistung am Kopf der Kondensationskolonne in selbige rückführen. Ein weiterer Teil des Sauerwassers wird zweckmäßigerweise ausgeschleust und entsorgt (z.B. verbrannt) und der verbleibende Teil des Sauerwassers wird üblicherweise in einem externen Wärmetauscher indirekt abgekühlt und als die Quenchflüssigkeit 3 verwendet. Leichter als Wasser flüchtige Bestandteile des Leichtsiederstroms bilden wiederum Abgas, das normalerweise wenigstens teilweise als Kreisgas in die Gasphasenoxidation rückgeführt bzw. bei der Rückspaltung mitverwendet wird.

Zweckmäßig erstrecken sich die Dual-Flow-Böden bei der bevorzugten Variante des erfindungsgemäßen Verfahrens in der Kondensationskolonne in etwa bis zu dem Querschnitt in der kondensationskolonne, von dem ab die Acrylsäuregehalte der Rücklaufflüssigkeit zum Kolonnenkopf hin betrachtet ≤ 90 Gew.-%, bezogen auf das Gewicht der Rücklaufflüssigkeit, betragen.

Die Anzahl an Dual-Flow-Böden beträgt, wie bereits gesagt, für die Vorzugsvariante des erfindungsgemäßen Verfahrens in der Regel 25 bis 45. Ihr Öffnungsverhältnis liegt zweckmäßig bei 15 bis 25 %. Als Durchtrittsstellen weisen die Dual-Flow-Böden dabei bevorzugt kreisrunde Löcher mit einem einheitlichen Kreisdurchmesser auf. Letzterer beträgt zweckmäßig 10 bis 20 mm. Bei Bedarf kann man in der Kondensationskolonne die Lochdurchmesser von oben nach unten verjüngen oder vergrößern und/oder die Anzahl der Löcher vermindern oder vergrößern (z.B. kann der Lochdurchmesser einheitlich 14 mm betragen und das Öffnungsverhältnis von oben nach unten von 17,4 % auf 18,3 % zunehmen). Jedoch kann die Lochanzahl auch über alle Dual-Flow-Böden konstant sein. Ferner sind die kreisrunden Löcher über den individuellen Dual-Flow-Böden bevorzugt in strenger Dreiecksteilung gleichmäßig angeordnet (vgl. DE-A 10230219).

Außerdem zeigt der Stanzgrat der in den Dual-Flow-Böden herausgestanzten Durchtrittsöffnungen in der Kondensationskolonne bevorzugt nach unten (unerwünschte Polymerisatbildung wird dadurch gemindert).

Erfindungsgemäß sinnvoll ist es, wenn die Anzahl der in der Kondensationskolonne eingesetzten Dual-Flow-Böden etwa 10 bis 15 theoretischen Trennstufen entspricht.

Die Anzahl der sich an die Dual-Flow-Böden in der erfindungsgemäß bevorzugten Kondensationskolonne anschließenden hydraulisch abgedichteten Querstrom-Stoffaustauschböden wird, wie bereits erwähnt, in der Regel 30 bis 50 betragen. Ihr Öffnungsverhältnis wird zweckmäßig 5 bis 25 %, bevorzugt 10 bis 20 % betragen (das Öffnungsverhältnis gibt ganz generell den prozentualen Anteil der Durchtrittsquerschnitte am Gesamtquerschnitt wieder; es liegt bei den vorzugsweise mitzuverwendenden Querstrom-Stoffaustauschböden ganz generell zweckmäßig im vorgenannten Bereich).

Einflutige Querstrom-Stoffaustauschböden werden erfindungsgemäß bevorzugt eingesetzt.

In der Regel wird die Anzahl der hydraulisch abgedichteten Querstromböden für die Vorzugsvariante des erfindungsgemäßen Verfahrens so bemessen, dass sie etwa 10 bis 30, häufig 25 theoretischen Trennstufen entspricht.

Sowohl die hydraulisch abgedichteten Querstromböden als auch gegebenenfalls mitverwendete Ventilböden weisen wenigstens einen Ablaufschacht auf. Sie können beide sowohl einflutig als auch mehrflutig, z.B. zweiflutig gestaltet sein, Dabei können sie auch bei einflutiger Gestaltung mehr als einen Ablaufschacht aufweisen. In der Regel sind auch die Zulaufschächte der Ventilböden hydraulisch abgedichtet.

Die Polymerisationsinhibierung des Quenchsystems 1 für das Produktgasgemisch der partiellen Gasphasenoxidation kann sowohl über in zum Quenchen verwendeter Sumpfflüssigkeit enthaltene Polymerisationsinhibitoren als auch über in zum Quenschen verwendeter Schwersiederfraktion enthaltene Polymerisationsinhibitoren bewerkstelligt werden.

Der Vorteil des erfindungsgemäßen Verfahrens liegt nochmals darin begründet, daß es bei gleicher Ausbeute eine erhöhte Reinheit der rohen Acrylsäure (und damit aller nachfolgenden Reinigungsstufen derselben) bzw. bei gleicher Reinheit eine erhöhte Ausbeute an roher Acrylsäure (und damit aller nachfolgenden Reinigungsstufen derselben) bedingt. Alle in dieser Schrift gemachten Aussagen gelten insbesondere für ein Produktgasgemisch, das durch heterogene Partialoxidation von Propylen zu Acrylsäure erhalten wurde. Die vorstehend beschriebene bevorzugte Ausführungsvariante des erfindungsgemäßen Verfahrens beschränkt in keinster Weise dessen allgemeine Ausführbarkeit.

Beispielsweise kann es auch wie in Figur 2 der DE-A 19924533 dargestellt oder wie in der DE-A 10235847 beschrieben durchgeführt werden, jedoch mit dem Unterschied, daß die Spaltgase vor ihrer Rückführung einer Gegenstrom-Rektifikation unterworfen werden.

### Beispiel und Vergleichsbeispiel

### 1. Beispiel (beschrieben wird der stationäre Zustand)

Aus einer heterogen katalysierten Gasphasenoxidation von Propylen der Reinheit "polymer grade" wurde ein eine Temperatur von 270°C aufweisendes Produktgasgemisch der nachfolgenden Zusammensetzung erhalten:
11,8201 Gew.-% Acrylsäure,
0,2685 Gew.-% Essigsäure,
5,2103 Gew.-% Wasser,
0,0279 Gew.-% Ameisensäure,
0,0989 Gew.-% Formaldehyd,
0,1472 Gew.-% Acrolein,
0,0028 Gew.-% Propionsäure,
0,0033 Gew.-% Furfurale,
0,0014 Gew.-% Allylacrylat,
0,0005 Gew.-% Allylformiat,
0,0038 Gew.-% Benzaldehyd,
0,1352 Gew.-% Maleinsäureanhydrid,
0,0113 Gew.-% Benzoesäure,
0,0147 Gew.-% Phthalsäureanhydrid,
1,8042 Gew.-% CO₂,
0,5898 Gew.-% CO,
0,5519 Gew.-% Propan,
0,2693 Gew.-% Propylen,
4,0253 Gew.-% O₂, und
75,0145 Gew.-% N₂.
Weitere Bestandteile werden nicht detektiert.

Das Produktgasgemisch (170855 kg/h) wird in einem im Gleichstrom betriebenen Sprühkühler (Quench 1) durch direkte Kühlung auf eine Temperatur von 113, 2°C abgekühlt.

Die zur Direktkühlung verwendete Flüssigkeit (Quenchflüssigkeit 1) ist ein Gemisch aus Sumpfflüssigkeit, die dem Sumpf der im folgenden beschriebenen Kondensationskolonne entnommen wird, und aus Schwersiederfraktion, die dem den Sumpfraum dieser Kondensationskolonne abschließenden ersten Fangboden entnommen wird.

Die Zusammensetzung der Sumpfflüssigkeit lautet:
41,0698 Gew.-% Acrylsäure,
0,2047 Gew.-% Essigsäure,
0,8436 Gew.-% Wasser,
0,0094 Gew.-% Ameisensäure,
0,0009 Gew.-% Formaldehyd,
0,0116 Gew.-% Acrolein,
0,0231 Gew.-% Propionsäure,
0,2317 Gew.-% Furfurale,
0,0011 Gew.-% Allylacrylat,
0,0001 Gew.-% Allylformiat
0,2506 Gew.-% Benzaldehyd,
6,1970 Gew.-% Maleinsäureanhydrid,
0,7396 Gew.-% Benzoesäure,
0,9658 Gew.-% Phthalsäureanhydrid,
21,0855 Gew.-% Diacrylsäure,
24,4047 Gew.-% Polyacrylsäure (Michael-Addukte),
0,4886 Gew.-% Phenothiazin,
0,6618 Gew.-% MEHQ,
0,0819 Gew.-% sonstige hochsiedende Bestandteile, und
0,0002 Gew.-% Sauerstoff.

Die Schwersiederfraktion weist folgende Zusammensetzung auf:
91,6313 Gew.-% Acrylsäure,
0,3777 Gew.-% Essigsäure,
1,4924 Gew.-% Wasser,
0,0145 Gew.-% Ameisensäure,
0,0016 Gew.-% Formaldehyd,
0,0108 Gew.-% Acrolein,
0,0528 Gew.-% Propionsäure,
0,4322 Gew.-% Furfurale,
0,0025 Gew.-% Allylacrylat,
0,0003 Gew.-% Allylformiat,
0,3321 Gew.-% Benzaldehyd,
4,4666 Gew.-% Maleinsäureanhydrid,
0,0193 Gew.-% Benzoesäure,
0,0169 Gew.-% Phthalsäureanhydrid,
1,0767 Gew.-% Diacrylsäure,
0,0175 Gew.-% Phenothiazin,
0,0544 Gew.-% MEHQ, und
0,0004 Gew.-% Sauerstoff.

Die Menge der entnommenen Schwersiederfraktion beträgt 72629 kg/h. Sie wird mit einer Temperatur von 99,5°C entnommen und mit dieser Temperatur dem Sprühkühler (Quench 1) zugeführt. Die Menge der der Kondensationskolonne entnommenen Sumpfflüssigkeit beträgt 249816 kg/h. Sie wird mit einer Temperatur von 110,8°C entnommen. Dem Sprühkühler wird mit dieser Temperatur nur eine Menge von 247216 kg/h zugeführt. 2300 kg/h werden der Rückspaltung zugeführt und 300 kg/h werden dem Quenchkreis 2 zugeführt, um diesen gegen unerwünschte Polymerisation zu inhibieren.

Das bei der Direktkühlung resultierende Gemisch aus auf 113, 2°C abgekühltem Produktgasgemisch und Quenchflüssigkeit 1 wird als solches in den Sumpf der Kondensationskolonne geführt. Der Druck im Sumpfraum und im Quench 1 beträgt 1,48 bar. Die Höhe der Kondensationskolonne beträgt 54,3 m.

Der Innendurchmesser der Kondensationskolonne beträgt im Bereich der Thormann-Böden 6,5 m und ansonsten 6,0 m.

2300 kg/h der entnommenen Sumpfflüssigkeit werden einer (Rück)spaltvorrichtung bestehend aus einem Zwangsumlaufentspannungsverdampfer und einer auf diesen nahtlos aufgesetzten Dual-Flow-Boden Rektifikationskolonne zugeführt. Die Anzahl der Dual-Flow-Böden beträgt 50. Ebenso wie die Kondensationskolonne ist die Reaktifikationskolonne gegen die Umgebung isoliert. Der Innendurchmesser der Rektifikationskolonne beträgt über alle Dual-Flow-Böden einheitlich 2,4 m. Ihre Höhe liegt bei 27 m. Die Dual-Flow-Böden sind in der Rektifikationskolonne äquidistant (400 mm) angeordnet. Ihr Öffnungsverhältnis beträgt einheitlich 12 %. Von unten nach oben betrachtet beträgt der Lochdurchmesser der ersten acht Dual-Flow-Böden einheitlich 25 mm (Lochanordnung entsprechend strenger Dreiecksteilung) und der Lochdurchmesser aller nachfolgenden Dual-Flow-Böden liegt einheitlich bei 14 mm (Lochanordnung ebenfalls entsprechend strenger Dreiecksteilung). Die Zufuhr der der Rückspaltung zu unterwerfenden Sumpfflüssigkeit erfolgt auf den achten Dual-Flow-Boden (von unten).

In den Zwangsumlaufverdampfer werden 25481 kg/h von am Kopf der Kondensationskolonne abgeführtem, nachfolgend überhitztem und komprimiertem Kreisgas (als Unterstützungsgas) zugeführt (Druck = 2,9 bar; Temperatur: 160°C).

Die Zusammensetzung des Kreisgases lautet:
0,2651 Gew.-% Acrylsäure,
0,0989 Gew.-% Essigsäure,
2,9333 Gew-.% Wasser,
0,0059 Gew.-% Ameisensäure,
0,1720 Gew.-% Acrolein,
0,0002 Gew.-% Propionsäure,
0,0002 Gew.-% Furfurale,
0,0013 Gew.-% Allylformiat,
4,7235 Gew.-% Sauerstoff,
2,1171 Gew.-% CO₂,
0,6921 Gew.-% CO,
0,6466 Gew.-% Propan,
0,3161 Gew.-% Propylen und
88,0277 Gew.-% Stickstoff.

Dem Zwangsumlaufentspannungsverdampfer werden stetig 558028 kg/h Flüssigphase mit einer Temperatur von 156,5°C entnommen. Davon werden 557457 kg/h mit einer Temperatur von 161,5°C in den Zwangsumlaufentspannungsverdampfer rückgeführt.

Die anderen 571 kg/h davon werden entgast und mit Methanol verdünnt der Rückstandsverbrennung zugeführt.

Die im Zwangsumlaufentspannungsverdampfer gebildeten Spaltgase werden durch das zugeführte Unterstützungsgas in die aufsitzende Reaktifikationskolonne gefördert und steigen in dieser in absteigender Rücklaufflüssigkeit auf.

Aus dem Kopf der Rektifikationskolonne wird in einer Menge von 39514 kg/h ein Gasgemisch (umfassend Kreisgas und Spaltgas) herausgeführt (Temp. = 94,4°C, Druck = 1,60 bar) und in einem im Gleichstrom betriebenen Sprühkühler (Quench 2) durch direkte Kühlung auf eine Temperatur von 59,9°C abgekühlt und partial kondensiert.

Das nach der Direktkühlung verbleibende Gasgemisch wird in einer Menge von 27510 kg/h und mit nachfolgender Zusammensetzung in den Sumpfraum der Kondensationskolonne (nicht getaucht) rückgeführt:
7,5140 Gew.-% Acrylsäure,
0,1106 Gew.-% Essigsäure,
2,7934 Gew.-% Wasser,
0,0064 Gew.-% Ameisensäure,
0,0001 Gew.-% Formaldehyd,
0,1603 Gew.-% Acrolein,
0,0024 Gew.-% Propionsäure,
0,0034 Gew.-% Furfurale,
0,0001 Gew.-% Allylacrylat,
0,0012 Gew.-% Allylformiat,
0,0003 Gew.-% Benzaldehyd,
0,0041 Gew.-% Maleinsäureanhydrid,
4,3751 Gew.-% Sauerstoff,
1,9610 Gew.-% CO₂,
0,6411 Gew.-% CO, - '
0,5989 Gew.-% Propan,
0,2928 Gew.-% Propylen und
81,5349 Gew.-% Stickstoff.

Als Quenchflüssigkeit 2 wird ein Gemisch aus 300 kg/h dem Sumpf der Kondensationskolonne entnommener Sumpfflüssigkeit und bei der Direktkühlung im Quench 2 gebildetem Kondensat verwendet. 128713 kg/h diese Gemisches werden durch indirekte Kühlung auf 32°C abgekühlt und im Sprühkühler 2 versprüht. 12304 kg/h desselben Gemisches werden mit einer Temperatur von 59,9°C auf den obersten Dual-Flow-Boden der Rektifikationskolonne als Rücklaufflüssigkeit rückgeführt.

Die Zusammensetzung der Quenchflüssigkeit 2 lautet:
92,9743 Gew.-% Acrylsäure,
0,6443-Gew.-% Essigsäure,
4,7794 Gew.-% Wasser,
0,0204 Gew.-% Ameisensäure,
0,0356 Gew.-% Acrolein,
0,0294 Gew.-% Propionsäure,
0,0506 Gew.-% Furfurale,
0,0007 Gew.-% Allylacrylat,
0,0020 Gew.-% Allylformiat,
0,0093 Gew.-% Benzaldehyd,
0,2050 Gew.-% Maleinsäureanhydrid,
0,0181 Gew.-% Benzoesäure,
0,0236 Gew.-% Phthalsäureanhydrid,
0,5143 Gew.-% Diacrylsäure,
0,5950 Gew.-% Polyacrylsäure (Michael-Addukte),
0,0119 Gew.-% Phenothiazin,
0,0163 Gew.-% MEHQ,
0,0685 Gew.-% sonstige hochsiedende Bestandteile, und
0,0012 Gew.-% Sauerstoff.

In den Sumpfraum der Kondensationskolonne ist ein Zentrifugaltropfenabscheider integriert, der verhindert, dass Tröpfchen der Sumpfflüssigkeit aus dem Sumpfraum heraus nach oben mitgerissen werden.

Der Sumpfraum der Kondensationskolonne ist, wie bereits erwähnt, auf einer Kolonnenhöhe (wie alle Höhen vom Sumpfboden aus gerechnet) von 7,80 m durch einen ersten Fangboden (Sammelboden; Kaminböden mit 16 etwa gleichverteilten bedachten Kaminen; Kamindurchmesser: 600 mm; Kaminhöhe: 1 m) abgeschlossen.

Der Sammelboden ist doppelwandig mit 2° Gefälle nach Innen und mit zentraler Abzugstasse und Abzugsstutzen (DN - 200) gestaltet. Der freie Gasquerschnitt beträgt ca. 30 %.

Von diesem ersten Fangboden werden, wie bereits erwähnt, 72629 kg/h Flüssigkeit entnommen und in den Sprühkühler 1 geführt.

Die Sumpftemperatur beträgt 110, 8°C. Der Druck liegt bei 1,48 bar.

2,0 m oberhalb des ersten Fangbodens befindet sich der erste von zunächst 15 Dual-Flow-Böden. Diese Dual-Flow-Böden (Lochdurchmesser einheitlich 14 mm, Lochanzahl einheitlich 33678, Öffnungsverhältnis einheitlich 18 %) sind äquidistant angebracht mit einem Bodenabstand von 380 mm. Die Durchtrittsöffnungen bestehen aus kreisrunden Öffnungen des einheitlichen Durchmessers von 14 mm, wobei der Stanzgrat in der Trennkolonne nach unten zeigt. Das Öffnungsverhältnis beträgt ca. 20 %. Die Anordnung der Mittelpunkte der Durchtrittskreise folgt einer strengen Dreiecksteilung. Der nächstliegende Abstand zweier Kreismittelpunkte liegt bei 30 mm.

Der fünfzehnte Dual-Flow-Boden ist als Verteilerboden gestaltet. Zu diesem Zweck enthält er zwei Einsteckrohre (DN - 150) mit 40 Auslaufbohrungen (Durchmesser: 15 mm) je Einsteckrohr.

Die erste Serie von Dual-Flow-Böden wird mit einem zweiten Fangboden (Sammelboden; Kaminboden mit 16 ca. gleichmäßig verteilten bedachten Kaminen; Kaminhöhe ca. 1,70 m, Zentrale Abzugstasse mit Abzugsstutzen (DN - 250), freier Gasquerschnitt von - 30 %) abgeschlossen, der 1,50 m oberhalb des letzten Dual-Flow-Bodens untergebracht ist.

Von diesem zweiten Fangboden wird bei 1,47 bar kontinuierlich rohe Acrylsäure mit einer Temperatur von 100,6°C entnommen, die wie folgt zusammengesetzt:
96,9126 Gew.-% Acrylsäure,
0,4500 Gew.-% Essigsäure,
1,5250 Gew.-% Wasser,
0,0137 Gew.-% Ameisensäure,
0,0015 Gew.-% Formaldehyd,
0,0088 Gew.-% Acrolein,
0,0638 Gew.-% Propionsäure,
0,2000 Gew.-% Furfurale,
0,0027 Gew.-% Allylacrylate,
0,0003 Gew.-% Allylformiat,
0,0467 Gew.-% Benzaldehyd,
0,2164 Gew.-% Maleinsäureanhydrid,
0,5281 Gew.-% Diacrylsäure,
0,0120 Gew.-% Phenothiazin,
0,0180 Gew.-% MEHQ, und
0,0004 Gew.-% Sauerstoff.

455993 kg/h der dem zweiten Fangboden entnommenen rohen Acrylsäure wird durch indirekten Wärmetausch auf 110,6°C erwärmt und unmittelbar unterhalb des dem zweiten Fangboden nach oben folgenden Dual-Flow-Bodens in die Kondensationskolonne rückgeführt.

91045 kg/h der dem zweiten Fangboden entnommenen rohen Acrylsäure werden mehrstufig durch indirekten Wärmetausch (bevorzugt wärmeintegriert gegen in die Kondensationskolonne rückzuführende Mutterlauge) auf eine Temperatur von 29°C abgekühlt. Dann werden der abgekühlten rohen Acrylsäure 1099 kg/h Wasser zugefügt. Das resultierende Gemisch wird durch nochmaligen indirekten Wärmeaustausch auf 20°C abgekühlt und dann in zwei bis drei Kühlscheibenkristallisatoren geführt. Dabei handelt es sich jeweils um einen Trog, in welchem 20 bis 24 gewischte kreisförmige Kühlplatten (die innerlich von einem Kühlmedium (Gemisch aus Wasser und Glykol; Glykolanteil = 10 bis 50 Gew.-%, bevorzugt 25 bis 35 Gew.-%) durchflossen werden) im äquidistanten Abstand von 20 bis 40 cm hintereinander hängend angeordnet sind (Plattendurchmesser typisch 2 bis 4 m, bevorzugt 2,5 bis 3 m). Das Kühlmedium wird dabei im Gegenstrom zum kristallisierenden Gemisch durch den Kristallisator von Kühlscheibe zu Kühlscheibe weitergereicht. Es kann aber auch aufgeteilt auf 2 bis 3 parallele Ströme über die Kühlplatten geführt werden. Die Eintrittstemperatur des Kühlmediums (der Sole) beträgt -2 bis +5°C. Die Austrittstemperatur liegt 2 bis 7°C höher. Durch das Wischen der Kühlplatten wird die Ausbildung einer Kristallschicht unterdrückt. Die angewässerte rohe Acrylsäure wird von hinten nach vorne kontinuierlich durch den Kristaller geführt (gepumpt oder überlaufgeregelt). Die einphasige angewässerte rohe Acrylsäure verdickt dabei (Verweilzeit 0,5 bis 4 h, bevorzugt 1,5 bis 2,5 h) zu einer zweiphasigen, Acrylsäurekristalle als feste Phase enthaltenden Suspension einer Temperatur von 6 bis 11°C und einem Feststoffgehalt am Austritt von 20 bis 35 Gew.-%. Die Drehzahl der Wischer beträgt 2 bis 15 Umdrehungen je Minute, bevorzugt 4 bis 10 Umdrehungen je Minute. Die die Wischer treibende, zentriert durch die Kühlscheiben geführte Welle, ist mit wassergespülten Stopfbuchspackungen (Packungsschnüre aus Teflon oder Graphit) abgedichtet.

Auf dem Umfang der Kühlscheiben, wo nicht gewischt werden kann, ist ein Hohlprofil (z.B. in einfachster Ausführungsform ein Rohr) aufgebracht (z.B. aufgeschweißt), das mittels eines zweiten Wärmeträgers (z.B. ebenfalls Wasser/Glykol Gemisch) beheizt wird (auf eine Temperatur oberhalb der Kristallisationstemperatur; meist aus dem Temperaturbereich 8 bis 20°C, bevorzugt 10 bis 14°C). Diese Umfangbeheizungen werden mit dem zweiten Wärmeträger parallel angeströmt.

Darüber hinaus sind die Wischer in radialer Richtung bevorzugt segmentiert (≥ 2, ≤ 6 Segmente in der Regel). Die spezifische Anpresskraft der Wischer liegt im eingebauten Zustand senkrecht zur Kühlfläche bei 1 bis 10, bevorzugt 3 bis 5 N pro cm aktiver Wischkantenlänge. Zusätzlich zu den Wischern treibt die Welle Paddel an (zwischen zwei Kühlscheiben und vor der ersten und letzten Kühlscheibe zweckmäßig jeweils zwei in symmetrischer Anordnung), die eine verbesserte Durchmischung bewirken.

Im in Förderrichtung der Suspension hinteren Teil des Kristallisators (bevorzugt hinter der letzten Kühlscheibe) wird die Suspension über ein angeschlossenes Rohr (zweckmäßigerweise getaucht angebracht; alternativ kann die Suspension über.ein Überlaufwehr in einen gerührten Sammelbehälter fließen, von dem aus die Waschkolonnen beschickt werden) zu hydraulischen Schmelze-Waschkolonnen geführt, wie sie in der DE-A 10156016 und in der DE-A 10223058 beschrieben sind, um die Mutterlauge vom Suspensionskristallisat abzutrennen. Die Beschickung der Waschkolonnen mit Kristallsuspension erfolgt mittels einer Kreisel- oder Kreiskolbenpumpe. Die Steuerstrompumpe ist ebenfalls als Kreiskolbenpumpe oder als Kreiselpumpe mit Regelventil ausgeführt. Der Druck am unteren Ende einer Waschkolonne ist üblicherweise ≥ 100 mbar und ≤5 bar kleiner als der Druck am Kopf der Waschkolonne. Der Kopfdruck beträgt in der Regel bis zu 6 bar, meist 0,5 bis 4 bar. Die Messerdrehzahl liegt meist bei Werten > 0 und ≤ 100/min, bzw. ≤ 60/min. Die Temperatur im Schmelzkreis beträgt normal 13 bis 16°C. Die Erfassung der Filtrationsfront wird gemäß der DE-A 10036880 über 2 bis 4 optische Sensoren vorgenommen. Die Waschfront wird mittels Temperaturmessung im Kristallbett geregelt.

Die Gesamthöhe des Kristallbetts liegt typisch bei 300 bis 1500 mm, meist bei 400 bis 1000 mm. Die Waschfront befindet sich typisch 10 bis 400 mm, meist bei 20 bis 250 mm oberhalb des Messers. Als Schmelzkreispumpe eignet sich eine Kreiselpumpe mit produktseitiger Spülung der Wellenabdichtung (Gleitringdichtung) oder eine magnetgekuppelte Pumpe mit erhöhter Gleitlagerspülung. Die Umlaufmenge im Schmelzkreis beträgt 2 bis 30; meist 5 bis 20 m³/h pro Tonne mit dem Messer abgetragenem gereinigtem Kristallisat. Die Stabilisierung des Schmelzkreises erfolgt mittels 100 bis 300 Gew.-ppm MEHQ. Zusätzlich wird in den Schmelzkreis Luft eingetragen, deren Überschuß (= nicht in der Waschschmelze gelöster Anteil) vor Eintritt der Waschschmelze in die Waschkolonne via Gasabscheider abgetrennt wird
[a) Zur Herstellung einer veresterungsgerechten Acrylsäure ist es ausreichend die Abtrennung des Suspensionskristallisats anstelle in einer Schmelze-Waschkolonne mittels einer Zentrifuge (z.B. einer 2- oder 3-stufigen Schubzentrifuge) durchzuführen. Geeignete Siebspaltweiten liegen bei 150 bis 300 µm; anwendbare Zentrifugalbeschleunigungen liegen bei 500 bis 900 g, meist 600 bis 800 g; geeignet sind Hubzahlen von 40 bis 80 Schüben/min..
   Vorzugsweise werden die auf der 2. oder 3. Stufe der Zentrifuge abgetrennten Kristalle mit 0,15 bis 0,3 kg Waschflüssigkeit pro kg Kristallisat gewaschen. Die Temperatur der Waschflüssigkeit liegt bei 15 bis 30°C, bevorzugt bei 20 bis 30°C. Zur Vermeidung von Ablagerungen wird der Feststoffabwurfschacht der Zentrifuge mit auf 15 bis 30°C temperierter Spülflüssigkeit gespült. Spül- und Waschflüssigkeit sind bevorzugt aufgeschmolzenes, über die Zentrifuge abgetrenntes und gewaschenes Kristallisat. Zur Vermeidung von Ablagerungen und Verkrustungen ist es zweckmäßig, das Zentrifugengehäuse, das Suspensionszuführrohr und das Waschflüssigkeitszuführrohr auf eine Temperatur ≥ 15°C und ≤ 40°C zu temperieren. Der Produktraum der Zentrifuge wird zweckmäßig mit Stickstoff oder mit einem Gemisch aus Luft und Stickstoff inertisiert. Die Wellenabdichtung wird mit Gas (z.B. Stickstoff oder ein Gemisch aus Luft und Stickstoff) oder mit Wasser gespült.
b) Alternativ zur Suspensionskristallisation kann auch eine Schichtkristallisation (z.B. Fallfilmkristallisation gemäß EP-A 616998 oder voll durchströmtes Rohr) mit 3 oder mehr (z.B. 3 bis 4) Reinigungsstufen angewendet werden. Anstelle die Mutterlauge einer nachgehenden Reinigungsstufe in eine vorgehende Reinigungsstufe rückzuführen, kann man sie auch gemeinsam in die Kondensationsskolonne rückführen.]

Den Schmelzkreisen, die durch den Zusatz von 3 kg/h MEHQ stabilisiert werden, werden 19584 kg/h an Reinacrylsäure der nachfolgenden Zusammensetzung entnommen:
99,8360 Gew-.% Acrylsäure,
0,0950 Gew.-% Essigsäure,
0,0332 Gew.-% Wasser,
0,0203 Gew.-% Propionsäure,
0,0001 Gew.-% Furfurale,
0,0001 Gew.-% Maleinsäureanhydrid,
0,0003 Gew.-% Diacrylsäure, und
0,0150 Gew.-% MEHQ.

Sie eignet sich in hervorrangender Weise zur Herstellung von Superabsorbern auf der Basis von Poly-Na-Acrylat.

In 834 kg/h der Reinacrylsäure werden 13 kg/h PTZ zur Herstellung einer Inhibitorlösung 1 gelöst. In 30 kg/h Inhibitorlösung 1 werden 19 kg/h MEHQ unter Bildung der Inhibitorlösung 2 gelöst.

Die in den Waschkolonnen abgetrennte Mutterlauge wird zunächst in einen heizbaren Sammelbehälter und von dort in einen Tank gefahren. Von diesem wird sie wärmeintegriert auf 90°C erwärmt in einer Menge von 72564 kg/h auf den fünfzehnten Dual-Flow-Boden der Kondensationskolonne (von unten gerechnet) rückgeführt. Die Zusammensetzung dieser rückgeführten Mutterlauge ist wie folgt:
94,6506 Gew.-% Acrylsäure,
0,0539 Gew.-% Essigsäure,
3,4196 Gew.-% Wasser,
0,0172 Gew.-% Ameisensäure,
0,0018 Gew.-% Formaldehyd,
0,0111 Gew.-% Acrolein,
0,0746 Gew.-% Propionsäure,
0,2509 Gew.-% Furfurale,
0,0034 Gew.-% Allylacrylat,
0,0003 Gew.-% Allylformiat,
0,0586 Gew.-% Benzaldehyd,
0,2715 Gew.-% Maleinsäureanhydrid,
0,6625 Gew.-% Diacrylsäure,
0,0151 Gew.-% Phenothiazin,
0,0233 Gew.-% MEHQ, und
0,0005 Gew.-% Sauerstoff.

2,9 m oberhalb des zweiten Fangbodens befindet sich in der Kondensationskolonne der erste von 21 weiteren Dual-Flow-Böden der bereits beschriebenen Art (Lochdurchmesser wieder einheitlich 14 mm, Lochanzahl jedoch einheitlich 32020 und Öffnungsverhältnis einheitlich 17,4 %), die wieder äquidistant mit einem Bodenabstand von 380 mm angeordnet waren. Der letzte dieser 21 Dual-Flow-Böden ist mit Überlaufrinnen mit gezacktem Überlauf als Verteilerboden gestaltet.

800 mm oberhalb des letzten Dual-Flow-Bodens beginnt sich die Kondensationskolonne konisch zu erweitern. 500 mm oberhalb des letzten Dual-Flow-Bodens endet diese Erweiterung bei einem Kolonneninnendurchmesser von 6,50 m.

Auf dieser Höhe, d.h., 1,50 m oberhalb des letzten Dual-Flow-Bodens, beginnt eine äquidistante (Bodenabstand = 500 mm) Anordnung von 28 konventionellen, einflutigen Thormann-Böden. Die Thormann-Böden sind derart ausgestaltet, dass über die Anordnung der Treibschlitze in den Hauben der Thormannn-Böden in in QuerstromRichtung aufeinanderfolgenden Rinnen jeweils eine zueinander entgegengesetzte Strömungsrichtung der Flüssigkeit erzeugt wird.

Das Öffnungsverhältnis der Thormann-Böden beträgt 14 %. Das Verhältnis von Kaminfläche zu Schlitzaustrittsfläche beträgt 0,8. Die Kaminhöhe und die Höhe des Ablaufwehrs beträgt 40 mm. Die Bodenfreiheit der Glocke (Abstand zwischen Unterkante Schlitz und Boden) beträgt 10 mm. Die Schlitzhöhe beträgt 15 mm. Der Winkel zwischen ausgestelltem Schlitz und Längskante der Haube beträgt 30 Grad. Die Länge der Längskante der Haube beträgt maximal 800 mm. Im Randbereich der Kolonne reduziert sich die Haubenlänge auf bis zu 200 mm aus Gründen der Anpassung an die Rundheit der Kolonne. Der Abstand zwischen zwei in Querstromrichtung auf einer Linie befindlichen Hauben beträgt 66 mm. Die Ablauffläche des Ablaufschachts beträgt 1,5 % bezogen auf die Querschnittsfläche des Bodens. Die Breite zwischen den beiden unteren Längsrändern einer Haube beträgt 64 mm.

Auf der Höhe des obersten Thormann-Bodens beginnt sich die Trennkolonne wieder konisch zu verengen. 700 mm oberhalb des obersten Thormann-Bodens ist diese Verengung abgeschlossen und der Kolonneninnendurchmesser wieder auf 6,00 m zurückgeschrumpft.

1,70 m oberhalb des obersten Thormann-Bodens befindet sich der dritte Fangboden (Sammelboden, Kaminboden mit 16 ca. gleichmäßig verteilten bedachten Kaminen, Kaminhöhe = 1,50 m).

Vom dritten Fangboden werden 533818 kg/h Sauerwasser mit einer Temperatur von 68,6°C und bei einem Druck von 1,24 bar entnommen.

Die Zusammensetzung des Sauerwassers ist:
11,8864 Gew.-% Acrylsäure,
4,1983 Gew.-% Essigsäure,
80,6473 Gew.-% Wasser,
0,5365 Gew.-% Ameisensäure,
2,3900 Gew.-% Formaldehyd,
0,0153 Gew.-% Acrolein,
0,0092 Gew.-% Propionsäure,
0,0016 Gew.-% Furfurale,
0,0132 Gew.-% Allylformiat, und
0,0001 Gew.-% MEHQ.

29443 kg/h des entnommenen Sauerwassers (68,6°C) wird zusammen mit der Inhibitorlösung 2 auf den obersten Thormann-Boden rückgeführt.

817 kg/h der Inhibitorlösung 1 werden (von unten betrachtet) auf den 19ten Thormann-Boden rückgeführt (mit einer Temperatur von 25°C und einem Druck von 1,10 bar).

7071 kg/h des entnommenen Sauerwassers werden der Verbrennung zugeführt.

298383 kg/h des entnommenen Sauerwassers werden mit einer Temperatur von 29°C auf den sechsten der nachfolgend zu beschreibenden Ventilböden (von unten gerechnet) rückgeführt.

198922 kg/h des entnommenen Sauerwassers werden mit einer Temperatur von 22,5°C auf den obersten der nachfolgend zu beschreibenden Ventilböden rückgeführt.

2300 mm oberhalb des dritten Fangbodens sind in der Kondensationskolonne in äquidistanter Anordnung (Bodenabstand = 500 mm) 11 zweiflutige Ventilböden angebracht. Die Höhe des Ablaufwehrs beträgt 35 mm. Das Öffnungsverhältnis liegt bei 18 % und die Summe der Ablaufflächen der Ablaufschächte von zwei aufeinanderfolgenden Ventilböden beträgt 10 % der Kolonnenquerschnittsfläche. Als Ventile wurden VV12-Ventile der Fa. Stahl, DE, Viernheim verwendet.

Der Druck am Kopf der Kolonne beträgt 1,2 bar.
Am Kolonnenkopf verlassen 171078 kg/h Abgas mit einer Temperatur von 35,2°C und der nachfolgenden Zusammensetzung die Trennkolonne:
0,2651 Gew.-% Acrylsäure,
0,0989 Gew.-% Essigsäure,
2,9333 Gew.-% Wasser,
0,0059 Gew.-% Ameisensäure,
0,1720 Gew.-% Acrolein,
0,0002 Gew.-% Propionsäure,
0,0002 Gew.-% Furfurale,
0,0013 Gew.-% Allylformiat,
2,1171 Gew.-% CO₂,
0,6921 Gew.-% CO,
0,6466 Gew.-% Propan,
0,3161 Gew.-% Propylen
4,7235 Gew.-% O₂, und
88,0277 Gew.-% N₂.

In einem indirekten Wärmetauscher wird das Abgas auf 38°C erwärmt und anschließend werden 96984 kg/h dieses Abgases über einen Kreisgasverdichter als Verdünnungsgas in die Gasphasenoxidation und in die Rückspaltung geführt und 47094 kg/h des Abgases werden der Verbrennung zugeführt.

### 2. Vergleichsbeispiel

Wie das Beispiel, auf den Kopf der Rektifikationskolonne wird jedoch keine Rücklaufflüssigkeit geführt, d.h., der Quenchkreis 2 ist geschlossen und das durch die Rektifikationskolonne gasförmig durchströmende Gemisch aus Spaltgas und Kreisgas wird direkt in den Sumpf der Kondensationskolonne geleitet. Die Kreisgasmenge wird auf 3900 kg/h reduziert, um die Spalttemperatur von 161,5°C beizubehalten. Die Wassermenge wird auf 1390 kg/h erhöht, um den Wassergehalt im Zulauf zur Suspensionskristallisation beizubehalten.

Bei gleichen Mengen ändert sich die Reinheit von über Seitenabzug entnommener roher Acrylsäure und in den Waschkolonnen abgetrennter Reinacrylsäure auf nachfolgende Spezifikationen:
- rohe Acrylsäure:: 90,8894 Gew.-% Acrylsäure,
0,4500 Gew.-% Essigsäure,
1,2067 Gew.-% Wasser,
0,0133 Gew.-% Ameisensäure,
0,0015 Gew.-% Formaldehyd,
0,0093 Gew.-% Acrolein,
0,0649 Gew.-% Propionsäure,
2,9306 Gew.-% Furfurale,
0,0027 Gew.-% Allylacrylat,
0,0001 Gew.-% Allylformiat,
3,2064 Gew.-% Benzaldehyd,
0,5816 Gew.-% Maleinsäureanhydrid,
0,6131 Gew.-% Diacrlysäure,
0,0120 Gew.-% Phenothiazin,
0,0180 Gew.-% MEHQ, und
0,0003 Gew.-% Sauerstoff.
- Reinacrylsäure:: 99,8331 Gew.-% Acrylsäure,
0,0947 Gew.-% Essigsäure,
0,0332 Gew.-% Wasser,
0,0206 Gew.-% Propionsäure,
0,0014 Gew.-% Furfurale,
0,0003 Gew.-% Maleinsäureanhydrid,
0,0003 Gew.-% Diacrlysäure,
0,0150 Gew.-% MEHQ, und
0,0015 Gew.-% Benzaldehyd.

## Patentansprüche

1. Verfahren zur Herstellung von Acrylsäure, bei dem man durch heterogen katalysierte Gasphasen-Partialoxidation wenigstens eines C₃-Vorläufers der Acrylsäure mit molekularem Sauerstoff an im festen Aggregatzustand befindlichen Katalysatoren bei erhöhter Temperatur ein Acrylsäure enthaltendes Produktgasgemisch erzeugt, die Temperatur des heißen, Acrylsäure enthaltenden Produktgasgemisches durch direkte Kühlung mit einer Quenchflüssigkeit 1 zunächst verringert und anschließend das abgekühlte, gegebenenfalls Teile an verdampfter Quenchflüssigkeit 1 enthaltende, Produktgasgemisch in eine mit trennwirksamen Einbauten ausgerüstete Kondensationskolonne leitet, innerhalb der Kondensationskolonne in sich selbst aufsteigen lässt, dabei fraktionierend kondensiert sowie im Seitenabzug als Zielprodukt eine rohe Acrylsäure aus der Kondensationskolonne entnimmt und aus dem Sumpf der Kondensationskolonne Acrylsäure-Oligomere enthaltende Sumpfflüssigkeit oder über einen unterhalb des Seitenabzugs für die rohe Acrylsäure gelegenen Seitenabzug Acrylsäure-Oligomere enthaltende Schwersiederfraktion oder ein Gemisch aus solcher Acrylsäure-Oligomere enthaltender Sumpfflüssigkeit und Schwersiederfraktion aus der Kondensationskolonne entnimmt und als Quenchflüssigkeit 1 verwendet, den beim Abkühlen des Produktgasgemisches nicht verdampften Teil der Quenchflüssigkeit 1, gegebenenfalls über den Sumpf oder über die Schwersiederentnahme der Kondensationskolonne oder über beide sowie gegebenenfalls über einen Wärmetauscher, im Kreis führt und als Auslass einen Teil der Quenchflüssigkeit 1 aus diesem Kreislauf ausschleust und einem Spaltgefäß zuführt sowie in selbigem die im Auslass der Quenchflüssigkeit 1 enthaltenen Acrylsäure-Oligomere bei erhöhter Temperatur in Acrylsäure rückspaltet und dabei aus der Flüssigphase gasförmig entweichende, Acrylsäure enthaltende, Spaltgase in den Kreislauf der Quenchflüssigkeit 1 oder in die Kondensationskolonne oder in den Kreislauf der Quenchflüssigkeit 1 und in die Kondensationskolonne rückführt, wobei diese Rückführung gasförmig, oder kondensiert, oder teilkondensiert erfolgen kann, **dadurch gekennzeichnet, dass** die Spaltgase vor ihrer Rückführung einer Gegenstrom-Rektifikation unterworfen werden.

2. Verfahren nach Anspruch 1, **dadurch gekennzeichnet, dass** der C₃-Vorläufer Propylen, oder Acrolein, oder ein Gemisch aus Propylen und Acrolein ist.

3. Verfahren nach Anspruch 1 oder 2, **dadurch gekennzeichnet, dass** die Gegenstrom-Rektifikation der Spaltgase in einer Rektifikationskolonne durchgeführt wird, die als trennwirksame Einbauten ausschließlich Dual-Flow-Böden enthält.

4. Verfahren nach einem der Ansprüche 1 bis 3, **dadurch gekennzeichnet, dass** die Rückspaltung der im Auslass der Quenchflüssigkeit 1 enthaltenen Acrylsäure-Oligomere ohne Zusatz eines Spaltkatalysators erfolgt.

5. Verfahren nach einem der Ansprüche 1 bis 4, **dadurch gekennzeichnet, dass** als Spaltgefäß ein Zwangsumlaufentspannungsverdampfer verwendet wird.

6. Verfahren nach einem der Ansprüche 1 bis 5, **dadurch gekennzeichnet, dass** die Rückspaltung der im Auslass der Quenchflüssigkeit 1 enthaltenen Acrylsäure-Oligomere bei einem Arbeitsdruck von 1 bar bis 2 bar durchgeführt wird.

7. Verfahren nach einem der Ansprüche 1 bis 6, **dadurch gekennzeichnet, dass** der Auslass der Quenchflüssigkeit 1 nicht unmittelbar in das Spaltgefäß sondern über die diesem aufgesetzte Rektifikationskolonne in das Spaltgefäß geführt wird.

8. Verfahren nach einem der Ansprüche 1 bis 7, **dadurch gekennzeichnet, dass** die Gegenstrom-Rektifikation der Spaltgase in einer Rektifikationskolonne erfolgt, die von einem Sauerstoff enthaltenden Gas durchströmt wird.

9. Verfahren nach einem der Ansprüche 1 bis 8, **dadurch gekennzeichnet, dass** das Spaltgefäß und die Rektifikationskolonne, in der die Gegenstrom-Rektifikation der Spaltgase erfolgt, von einem Sauerstoff enthaltenden Gas durchströmt werden.

10. Verfahren nach Anspruch 8 oder 9, **dadurch gekennzeichnet, dass** das Sauerstoff enthaltende Gas Kreisgas ist.

11. Verfahren nach einem der Ansprüche 1 bis 10, **dadurch gekennzeichnet, dass** die Rücklaufflüssigkeit für die Gegenstrom-Rektifikation der Spaltgase durch direkte Kühlung der Spaltgase erzeugt wird.

12. Verfahren nach einem der Ansprüche 1 bis 11, **dadurch gekennzeichnet, dass** zum Zweck der Polymerisationsinhibierung der Gegenstrom-Rektifikation der Spaltgase der Rücklaufflüssigkeit Polymerisationsinhibitor enthaltende Quenchflüssigkeit 1 zugesetzt wird.

13. Verfahren nach Anspruch 12, **dadurch gekennzeichnet, dass** als Polymerisationsinhibitor para-Methoxyphenol, oder Phenothiazin, oder ein Gemisch aus para-Methoxyphenol und Phenothiazin verwendet wird.

14. Verfahren nach einem der Ansprüche 1 bis 13, **dadurch gekennzeichnet, dass** die Rückführung der Spaltgase nach ihrer Gegenstrom-Rektifikation gasförmig in den Sumpfraum der Kondensationskolonne erfolgt.

15. Verfahren nach einem der Ansprüche 1 bis 14, **dadurch gekennzeichnet, dass** die Kondensationskolonne eine solche ist, die von unten nach oben, zunächst Dual-Flow-Böden und daran anschließend hydraulisch abgedichtete Querstromböden als trennwirksame Einbauten enthält.

16. Verfahren nach einem der Ansprüche 1 bis 15, **dadurch gekennzeichnet, daß** das, gegebenenfalls Teile an verdampfter Quenchflüssigkeit 1 enthaltende, Produktgasgemisch in den Sumpfraum der Kondensationskolonne und der beim Abkühlen des Produktgasgemisches nicht verdampfte Teil der Quenchflüssigkeit 1 über den Sumpf der Kondensationskolonne im Kreis geführt wird.

17. Verfahren nach einem der Ansprüche 1 bis 16, **dadurch gekennzeichnet, dass** die über Seitenabzug aus der Kondensationskolonne entnommene rohe Acrylsäure kristallisativ weitergereinigt und von der dabei anfallenden Mutterlauge in die Kondensationskolonne rückgeführt wird.

18. Verfahren nach Anspruch 17, **dadurch gekennzeichnet, dass** die Kristallisation eine Suspensionskristallisation ist.

19. Verfahren nach Anspruch 18, **dadurch gekennzeichnet, dass** das Suspensionskristallisat in einer Waschkolonne von der Mutterlauge abgetrennt wird.

## Claims

1. A process for preparing acrylic acid by generating an acrylic acid-comprising product gas mixture by heterogeneously catalyzed gas phase partial oxidation of at least one C₃ precursor of acrylic acid with molecular oxygen over catalysts in the solid state at elevated temperature, initially reducing the temperature of the hot acrylic acid-comprising product gas mixture by direct cooling with a quench liquid 1 and subsequently passing the cooled product gas mixture which may if appropriate comprise portions of evaporated quench liquid 1 into a condensation column equipped with separating internals, allowing it to rise into itself within the condensation column, thus fractionally condensing it, and withdrawing crude acrylic acid as the target product from the condensation column in a sidestream and withdrawing bottom liquid comprising acrylic acid oligomers from the bottom of the condensation column or removing high boiler fraction comprising acrylic acid oligomers or a mixture of such bottom liquid comprising acrylic acid oligomers and high boiler fraction from the condensation column via a sidestream takeoff disposed below the sidestream takeoff for the crude acrylic acid and using it as a quench liquid 1, recirculating the portion of the quench liquid 1 which is not evaporated on cooling the product gas mixture, if appropriate via the bottom or via the high boiler takeoff of the condensation column or via both and also if appropriate via a heat exchanger, discharging a portion of the quench liquid 1 from this circuit as a bleed stream and feeding it to a dissociation vessel and dissociating therein the acrylic acid oligomers present in the bleed stream of the quench liquid 1 at elevated temperature to acrylic acid, and recycling the dissociation gases comprising acrylic acid and escaping from the liquid phase in gaseous form into the circuit of the quench liquid 1 or into the condensation column or into the circuit of the quench liquid 1 and into the condensation column, either in gaseous, condensed or partially condensed form, which comprises subjecting the dissociation gases to a countercurrent rectification before they are recycled.

2. The process according to claim 1, wherein the C₃ precursor is propylene, or acrolein, or a mixture of propylene and acrolein.

3. The process according to claim 1 or 2, wherein the countercurrent rectification of the dissociation gases is carried out in a rectification column which comprises exclusively dual-flow trays as separating internals.

4. The process according to any of claims 1 to 3, wherein the acrylic acid oligomers comprised in the bleed stream of the quench liquid 1 are dissociated without the addition of a dissociation catalyst.

5. The process according to any of claims 1 to 4, wherein the dissociation vessel used is a forced circulation-decompression evaporator.

6. The process according to any of claims 1 to 5, wherein the dissociation of the acrylic acid oligomers comprised in the bleed stream of the quench liquid 1 is carried out at a working pressure of from 1 to 2 bar.

7. The process according to any of claims 1 to 6, wherein the bleed stream of the quench liquid 1 is not conducted immediately into the dissociation vessel, but rather conducted into the dissociation vessel via the rectification column attached thereto.

8. The process according to any of claims 1 to 7, wherein the countercurrent rectification of the dissociation gases is effected in a rectification column which is flowed through by an oxygen-comprising gas.

9. The process according to any of claims 1 to 8, wherein the dissociation vessel and the rectification column in which the countercurrent rectification of the dissociation gases is effected are flowed through by an oxygen-comprising gas.

10. The process according to claim 8 or 9, wherein the oxygen-comprising gas is cycle gas.

11. The process according to any of claims 1 to 10, wherein the reflux liquid for the countercurrent rectification of the dissociation gases is generated by direct cooling of the dissociation gases.

12. The process according to any of claims 1 to 11, wherein, for the purposes of polymerization inhibition of the countercurrent rectification of the dissociation gases, quench liquid 1 comprising polymerization inhibitor is added to the reflux liquid.

13. The process according to claim 12, wherein the polymerization inhibitor used is para-methoxyphenol, or phenothiazine, or a mixture of para-methoxyphenol and phenothiazine.

14. The process according to any of claims 1 to 13, wherein the dissociation gases, after their countercurrent rectification, are recycled into the bottom space of the condensation column in gaseous form.

15. The process according to any of claims 1 to 14, wherein the condensation column is one whose separating internals, from bottom to top, are initially dual-flow trays followed by hydraulically sealed crossflow trays.

16. The process according to any of claims 1 to 15, wherein the product gas mixture which may if appropriate comprise portions of evaporated quench liquid 1 is recirculated into the bottom space of the condensation column and the portion of the quench liquid 1 which does not evaporate when cooling the product gas mixture is recirculated via the bottom of the condensation column.

17. The process according to any of claims 1 to 16, wherein the crude acrylic acid removed from the condensation column via the sidestream takeoff is further purified crystallatively and the resulting mother liquid is recycled into the condensation column.

18. The process according to claim 17, wherein the crystallization is a suspension crystallization.

19. The process according to claim 18, wherein the suspension crystals are removed from the mother liquor in a washing column.

## Revendications

1. Procédé de préparation d'acide acrylique, dans lequel on produit un mélange gazeux produit contenant de l'acide acrylique par oxydation partielle en phase gazeuse à catalyse hétérogène d'au moins un précurseur en C₃ de l'acide acrylique avec de l'oxygène moléculaire sur des catalyseurs, qui se trouvent à l'état d'agrégats solides, à une température élevée, on diminue tout d'abord la température du mélange gazeux produit chaud, contenant de l'acide acrylique, par refroidissement direct avec un liquide de refroidissement brusque 1 et ensuite on conduit le mélange gazeux produit refroidi, contenant éventuellement des parties de liquide de refroidissement brusque 1 évaporées, dans une colonne de condensation équipée d'éléments encastrés actifs pour une séparation, on le laisse monter par soi-même à l'intérieur de la colonne de condensation, on condense de manière fractionnée et, dans un soutirage latéral, on prélève de la colonne de condensation, comme produit visé, un acide acrylique brut et on prélève de la colonne de condensation, à partir du fond de celle-ci, un liquide de fond contenant des oligomères d'acide acrylique ou, par l'intermédiaire d'un soutirage latéral situé en dessous du soutirage latéral de l'acide acrylique brut, une fraction à ébullition difficile contenant des oligomères d'acide acrylique ou un mélange d'un tel liquide de fond et d'une telle fraction à ébullition difficile contenant des oligomères d'acide acrylique et on l'utilise comme liquide de refroidissement brusque 1, on met en circuit la partie du liquide de refroidissement brusque 1 non évaporée lors du refroidissement du mélange gazeux produit, éventuellement par l'intermédiaire du fond ou du prélèvement à ébullition difficile de la colonne de condensation ou par l'intermédiaire des deux ainsi qu'éventuellement par un échangeur de chaleur, et on évacue par éclusage, hors de ce circuit, comme décharge, une partie du liquide de refroidissement brusque 1 et on l'amène à un récipient de craquage et dans celui-ci on recraque les oligomères d'acide acrylique contenus dans la décharge du liquide de refroidissement brusque 1 en acide acrylique, à température élevée, et on reconduit des gaz de craquage contenant de l'acide acrylique, qui se dégagent sous forme gazeuse hors de la phase liquide, dans le circuit du liquide de refroidissement brusque 1 ou dans la colonne de condensation ou dans le circuit du liquide de refroidissement brusque 1 et dans la colonne de condensation, ce recyclage pouvant avoir lieu sous forme gazeuse, ou condensée, ou partiellement condensée, **caractérisé en ce que** les gaz de craquage sont, avant leur recyclage, soumis à une rectification en contre-courant.

2. Procédé suivant la revendication 1, **caractérisé en ce que** le précurseur en C₃ est du propylène ou de l'acroléine ou un mélange de propylène et d'acroléine.

3. Procédé suivant la revendication 1 ou 2,
**caractérisé en ce que** la rectification en contre-courant des gaz de craquage est effectuée dans une colonne de rectification qui, comme éléments encastrés actifs pour une séparation, contient exclusivement des plateaux à deux passes.

4. Procédé suivant l'une des revendications 1 à 3, **caractérisé en ce que** le recraquage des oligomères d'acide acrylique contenus dans la décharge du liquide de refroidissement brusque 1 a lieu sans addition d'un catalyseur de craquage.

5. Procédé suivant l'une des revendications 1 à 4, **caractérisé en ce que**, comme récipient de craquage, on utilise un dispositif de vaporisation instantanée à circulation forcée.

6. Procédé suivant l'une des revendications 1 à 5, **caractérisé en ce que** le recraquage des oligomères d'acide acrylique contenus dans la décharge du liquide de refroidissement brusque 1 est effectué à une pression de travail de 1 bar à 2 bars.

7. Procédé suivant l'une des revendications 1 à 6, **caractérisé en ce que** la décharge du liquide de refroidissement brusque 1 n'est pas conduite immédiatement dans le récipient de craquage mais au contraire par l'intermédiaire de la colonne de rectification montée sur celui-ci.

8. Procédé suivant l'une des revendications 1 à 7, **caractérisé en ce que** la rectification en contre-courant des gaz de craquage a lieu dans une colonne de rectification qui est traversée par un gaz contenant de l'oxygène.

9. Procédé suivant l'une des revendications 1 à 8, **caractérisé en ce que** le récipient de craquage et la colonne de rectification, dans laquelle la rectification à contre-courant des gaz de craquage a lieu, sont traversés par un gaz contenant de l'oxygène.

10. Procédé suivant la revendication 8 ou 9, **caractérisé en ce que** le gaz contenant de l'oxygène est un gaz de circulation.

11. Procédé suivant l'une des revendications 1 à 10, **caractérisé en ce que** le liquide de reflux pour la rectification en contre-courant des gaz de craquage est produit par refroidissement direct des gaz de craquage.

12. Procédé suivant l'une des revendications 1 à 11, **caractérisé en ce que**, en vue de l'inhibition de polymérisation de la rectification en contre-courant des gaz de craquage, on ajoute au liquide de reflux du liquide de refroidissement brusque 1 contenant un inhibiteur de polymérisation.

13. Procédé suivant la revendication 12, **caractérisé en ce que**, comme inhibiteur de polymérisation, on utilise du para-méthoxyphénol ou de la phénothiazine ou un mélange de para-méthoxyphénol et de phénothiazine.

14. Procédé suivant l'une des revendications 1 à 13, **caractérisé en ce que** le recyclage des gaz de craquage après leur rectification en contre-courant a lieu sous forme gazeuse dans l'espace de fond de la colonne de condensation.

15. Procédé suivant l'une des revendications 1 à 14, **caractérisé en ce que** la colonne de condensation est une colonne qui contient, comme éléments encastrés actifs pour une séparation, de bas en haut, tout d'abord des plateaux à double passe et ensuite des plateaux à écoulement transversal étanchéifiés du point de vue hydraulique.

16. Procédé suivant l'une des revendications 1 à 15, **caractérisé en ce que** le mélange gazeux produit, contenant éventuellement des parties de liquide de refroidissement brusque 1 évaporées, est conduit dans l'espace de fond de la colonne de condensation et la partie du liquide de refroidissement brusque 1, qui n'a pas été évaporée lors du refroidissement du mélange gazeux produit, est conduite en circuit par l'intermédiaire du fond de la colonne de condensation.

17. Procédé suivant l'une des revendications 1 à 16, **caractérisé en ce que** l'acide acrylique brut prélevé de la colonne de condensation par un soutirage latéral est purifié davantage par cristallisation et est recyclé, à partir de la lessive mère obtenue, dans la colonne de condensation.

18. Procédé suivant la revendication 17, **caractérisé en ce que** la cristallisation est une cristallisation en suspension.

19. Procédé suivant la revendication 18, **caractérisé en ce que** le produit de cristallisation en suspension est séparé de la lessive mère dans une colonne de lavage.
